# EUROPEAN PATENT APPLICATION

(11) **EP 4 252 752 A1**
(43) Date of publication of application: **04.10.2023**
(21) Application number: 20963634.9
(22) Date of filing: 30.11.2020
(51) Int. Cl.: A61K 31/407, A61K 31/501, A61P 7/04, C07D 491/052, C07D 519/00

(54) **DRUG FOR TREATMENT OR PREVENTION OF CEREBRAL HEMORRHAGE, AND METHOD FOR TREATMENT OR PREVENTION OF CEREBRAL HEMORRHAGE USING SAID DRUG**

(71) Applicant: Biogen MA Inc., Cambridge, MA 02142 (US)
(72) Inventor: TOMINAGA, Teiji, Sendai-shi, Miyagi 980-8574 (JP); NIIZUMA, Kuniyasu, Sendai-shi, Miyagi 980-8574 (JP)
(74) Representative: Beck Greener LLP
(86) International application number: PCT/JP2020/044574
(87) International publication number: WO 2022/113371

(57) **Abstract**

Provided is a drug used for treatment or prevention of cerebral hemorrhage, containing as an active ingredient a compound of formula (I) below or a pharmaceutically acceptable salt, ester, or solvate thereof.

## Description

### Technical Field

The present disclosure is related to a drug for treating or preventing cerebral hemorrhage and a method of treating or preventing cerebral hemorrhage using said drug.

### Background Art

Cerebral hemorrhage is a general term for a condition in which a hemorrhage is caused by a blood vessel in the brain rupturing, and can be divided into intracerebral hemorrhage, subarachnoid hemorrhage, and the like depending on the hemorrhage site. Blood leaking from blood vessels may form a hematoma, and the hematoma directly damages the brain, or pressure in the brain may rise due to formation of an edema, and the brain being pressured causes damage to the brain.

Until now, no effective pharmacological treatment method had been established for cerebral hemorrhage, and removal of hematomas in the brain by surgery and the like has been used as a treatment.

Meanwhile, SMTP (*stachybotrys microspora* triprenyl phenol) compounds are a group of compounds having a triprenyl phenol structure produced by a filamentous fungus, and are known to have a thrombolysis promoting effect and angiogenesis inhibiting effect according to JP 2004-224737 A, JP 2004-224738 A, and WO 2007/111203. Regarding a thrombolysis promoting effect, according to FEBS Letter 1997; 418: 58-62, a mechanism of action is suggested wherein SMTP compounds induce conformational changes in plasminogen, resulting in increased susceptibility to t-PA of plasminogen and increased binding of plasminogen to thrombi, and the like, promoting dissolution of thrombi. Furthermore, according to J Biol Chem 2014; 289: 35826-35838, SMTP compounds have also been shown to have an excellent anti-inflammatory effect.

### Summary of Invention

### Problem to Be Solved by Invention

The present inventors have discovered that a compound of formula (I) or a pharmaceutically acceptable salt, ester, or solvate thereof has an effect in treatment or prevention of cerebral hemorrhage.

It is surprising that, while there have been no effective pharmacological treatment methods for cerebral hemorrhage up until now, a compound of formula (I) or a pharmaceutically acceptable salt, ester, or solvate thereof is effective in treatment or prevention of cerebral hemorrhage.

JP 2004-224737 A, JP 2004-224738 A, and WO 2007/111203, FEBS Letter 1997; 418: 58-62, and J Biol Chem 2014; 289: 35826-35838 do not teach or suggest an effect of a compound of formula (I) on cerebral hemorrhage.

A problem to be solved by the embodiments of the present disclosure is to provide a drug excellent for treatment or prevention of cerebral hemorrhage and a new treatment method or prevention method of cerebral hemorrhage.

### Means for Solving Problem

Means for solving the above problem includes the following aspects:
<1> A drug used for treatment or prevention of cerebral hemorrhage, containing as an active ingredient a compound of formula (I) below or a pharmaceutically acceptable salt, ester, or solvate thereof. 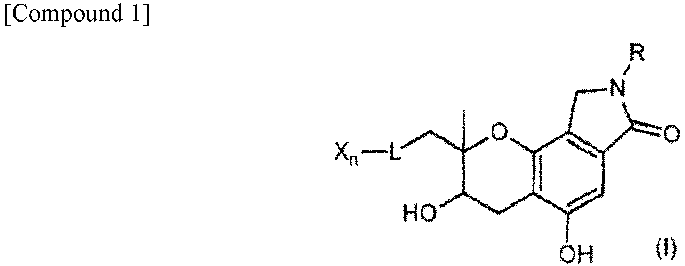 In formula (I), L represents an aliphatic hydrocarbon group having a carbon number of 4 to 10, X represents a hydroxy group or carboxy group, n represents an integer from 0 to 2, and R represents a hydrogen atom or a substituent having a molecular weight of 1,000 or less.
<2> The drug according to <1>, wherein the compound of the formula (I) or a pharmaceutically acceptable salt, ester, or solvate thereof is a compound of formula (IA) below or a pharmaceutically acceptable salt, ester, or solvate thereof. 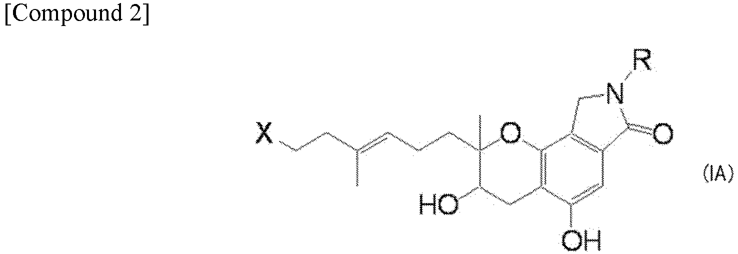 In formula (IA), X is -CHY-C(CH₃)₂Z, Y and Z are independently -H or -OH, or together form a single bond, and R represents a hydrogen atom or a substituent having a molecular weight of 1,000 or less.
<3> The drug according to <1> or <2>, wherein the compound of the formula (I) or a pharmaceutically acceptable salt, ester, or solvate thereof is a compound of formula (II) or formula (III) below or a pharmaceutically acceptable salt, ester, or solvate thereof. 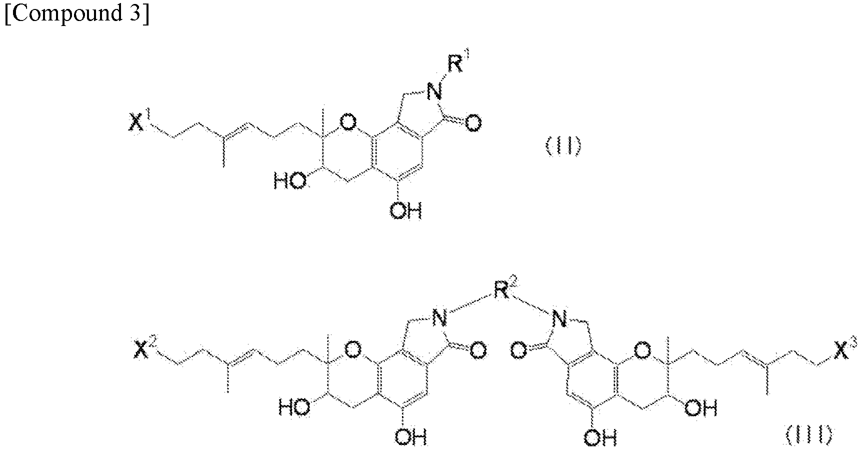 In formula (II) or formula (III), X¹, X², and X³ are independently -CHY-C(CH₃)₂Z, Y and Z are independently -H or -OH, or together form a single bond, and R¹ represents any one of (A) to (D) below.
   (A) A residue having one amino group removed from an amino compound selected from a group composed of natural amino acids, D-bodies of natural amino acids, and compounds having at least one carboxy group in a natural amino acid or D-body of a natural amino acid replaced with a hydrogen atom, a hydroxy group, or a hydroxymethyl group (however, -(CH)₂-OH is excluded),
   (B) an aromatic group having at least one selected from a group composed of a carboxy group, a hydroxy group, a sulfonic acid group, and a secondary amino group as a substituent or a part of a substituent, or an aromatic group containing a secondary amino group and that may contain a nitrogen atom,
   (C) an aromatic amino acid residue of formula (II-1) below (in the formula, R³ is a respectively independent substituent which may be present or absent, and if present, represents a hydroxy group, a carboxy group or an alkyl group having a carbon number of 1 to 5, n represents an integer of 0 or 1, m represents an integer from 0 to 5, and * represents a binding site), and 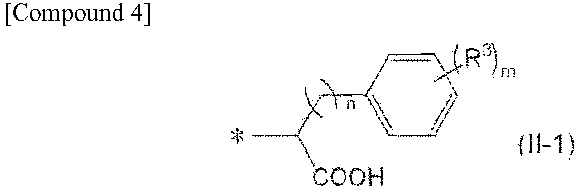
   (D) a substituent represented by -L¹-L²-R⁴ (in the formula, L¹ represents a linking group which is an alkylene group having a carbon number of 1 to 4 having a carboxy group, L² represents a linking group represented by -NH-C(=O)- or -NH-C(=S)-NH-, R⁴ represents a 9-fluorenyl alkyloxy group having an alkyloxy group having a carbon number of 1 to 3, or a polyheterocyclic group of formula (II-2) below (in formula (II-2), and * represents a binding site)). 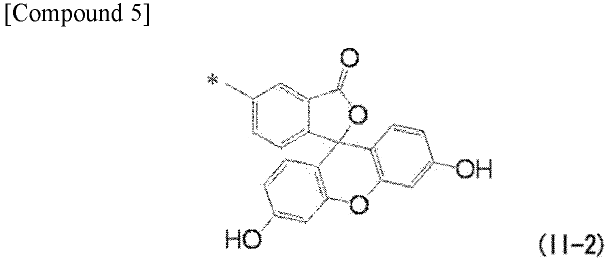 R² is a residue having two amino groups removed from an amino compound selected from a group composed of natural amino acids having 2 amino groups, D-bodies of natural amino acids having 2 amino groups, compounds having at least one carboxy group in a natural amino acid having 2 amino groups or a D-body of a natural amino acid having 2 amino groups replaced with a hydrogen atom, a hydroxy group, or a hydroxymethyl group, and compounds indicated by H₂N-CH(COOH)-(CH₂)ₙ-NH₂ (n is an integer from 0 to 9), and H₂N-CH(COOH)-(CH₂)ₘ-Sₚ-(CH₂)_{q}-CH(COOH)-NH₂ (m, p, and q are each independent integers from 0 to 9).
<4> The drug according to any one of <1> to <3>, wherein the compound of the formula (I) or a pharmaceutically acceptable salt, ester, or solvate thereof is a compound selected from a group composed of SMTP-0 below, SMTP-1 below, SMTP-4 below, SMTP-5D below, SMTP-6 below, SMTP-7 below, SMTP-8 below, SMTP-11 to 14 below, SMTP-18 to 29 below, SMTP-36 below, SMTP-37 below, SMTP-42 below, SMTP-43 below, SMTP-43D below, SMTP-44 below, SMTP-44D below, SMTP-46 below, and SMTP-47 below, or a pharmaceutically acceptable salt, ester, or solvate thereof. 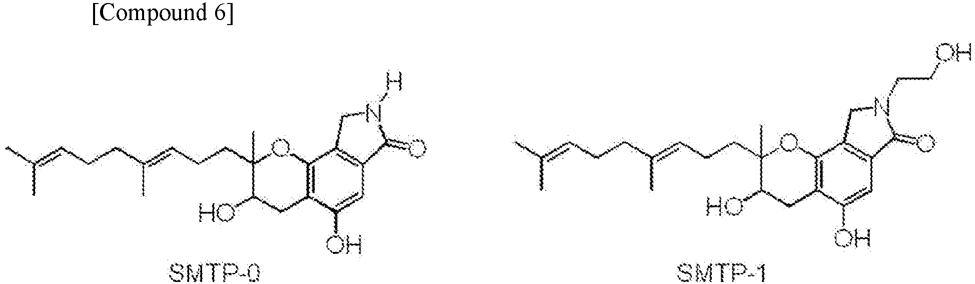 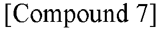 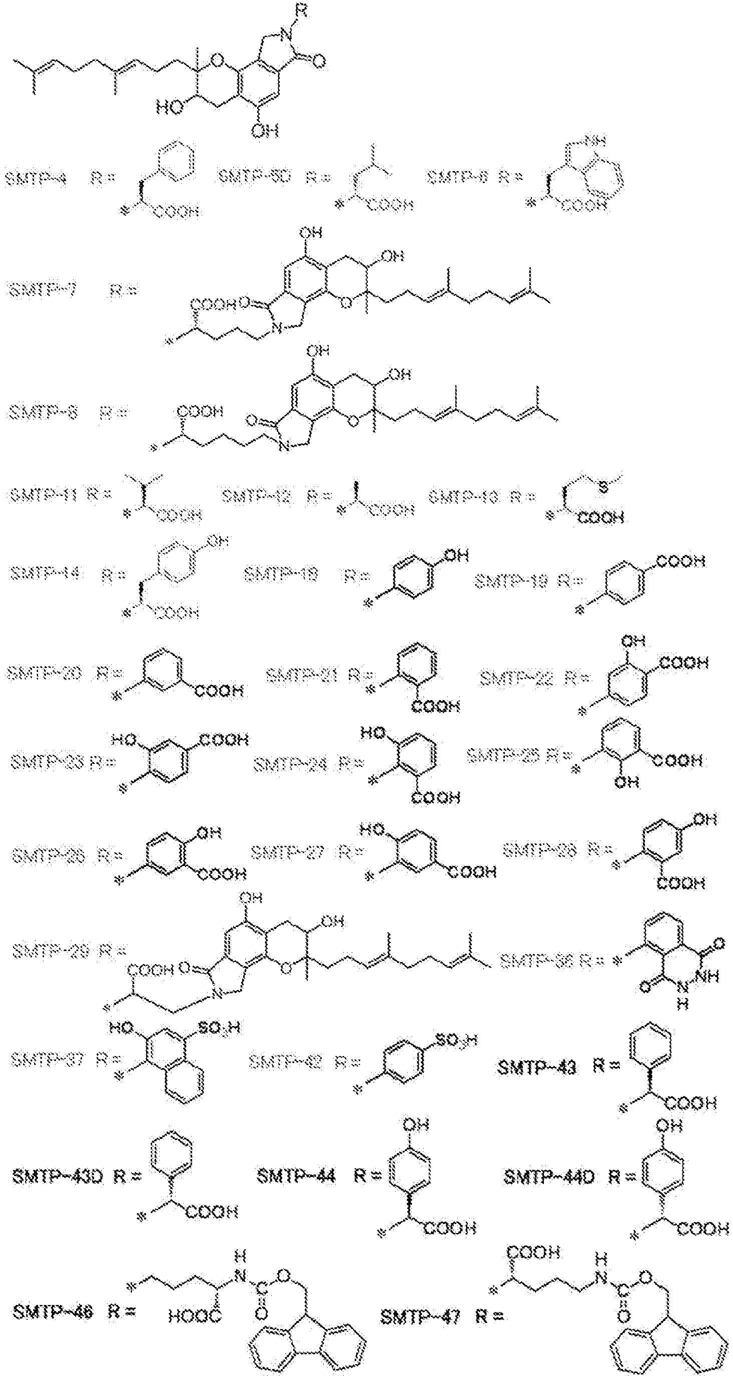 In the formula, * represents a binding site.
<5> The drug according to <4>, wherein the compound of the formula (I) or a pharmaceutically acceptable salt, ester, or solvate thereof is the SMTP-7 or a pharmaceutically acceptable salt, ester, or solvate thereof.
<6> The drug according to any one of <1> to <5>, wherein the cerebral hemorrhage is an intracerebral hemorrhage.
<7> The drug according to any one of <1> to <6>, which is intracerebrally administered.
<8> A method of treating cerebral hemorrhage in a subject including administering to the subject having cerebral hemorrhage a compound of formula (I) below or a pharmaceutically acceptable salt, ester, or solvate thereof at an amount effective for treating cerebral hemorrhage. 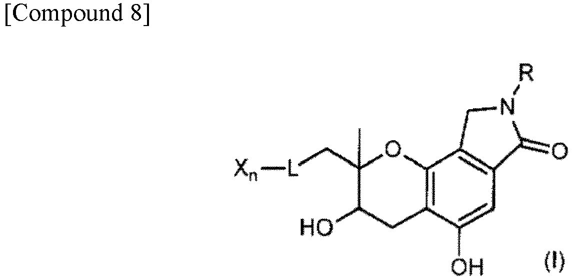 In formula (I), L represents an aliphatic hydrocarbon group having a carbon number of 4 to 10, X represents a hydroxy group or carboxy group, n represents an integer from 0 to 2, and R represents a hydrogen atom or a substituent having a molecular weight of 1,000 or less.
<9> A method of preventing cerebral hemorrhage in a subject including administering to the subject having a risk of onset of cerebral hemorrhage a compound of formula (I) below or a pharmaceutically acceptable salt, ester, or solvate thereof at an amount effective for preventing cerebral hemorrhage. 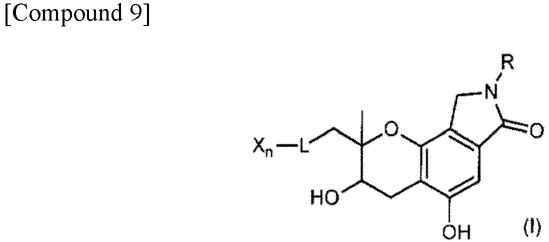 In formula (I), L represents an aliphatic hydrocarbon group having a carbon number of 4 to 10, X represents a hydroxy group or carboxy group, n represents an integer from 0 to 2, and R represents a hydrogen atom or a substituent having a molecular weight of 1,000 or less.
<10> The method according to <8> or <9>, wherein the compound of the formula (I) or a pharmaceutically acceptable salt, ester, or solvate thereof is a compound of formula (IA) below or a pharmaceutically acceptable salt, ester, or solvate thereof. 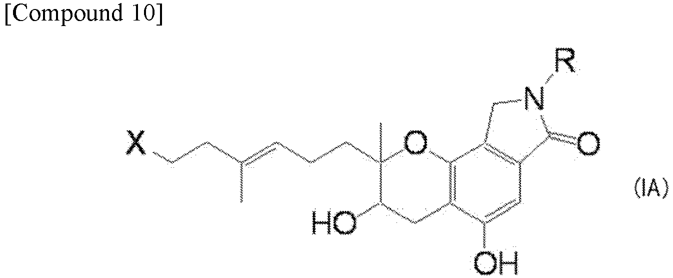 In formula (IA), X is -CHY-C(CH₃)₂Z, Y and Z are independently -H or -OH, or together form a single bond, and R represents a hydrogen atom or a substituent having a molecular weight of 1,000 or less.
<11> The method according to any one of <8> to <10>, wherein the compound of the formula (I) or a pharmaceutically acceptable salt, ester, or solvate thereof is a compound of formula (II) or formula (III) below or a pharmaceutically acceptable salt, ester, or solvate thereof. 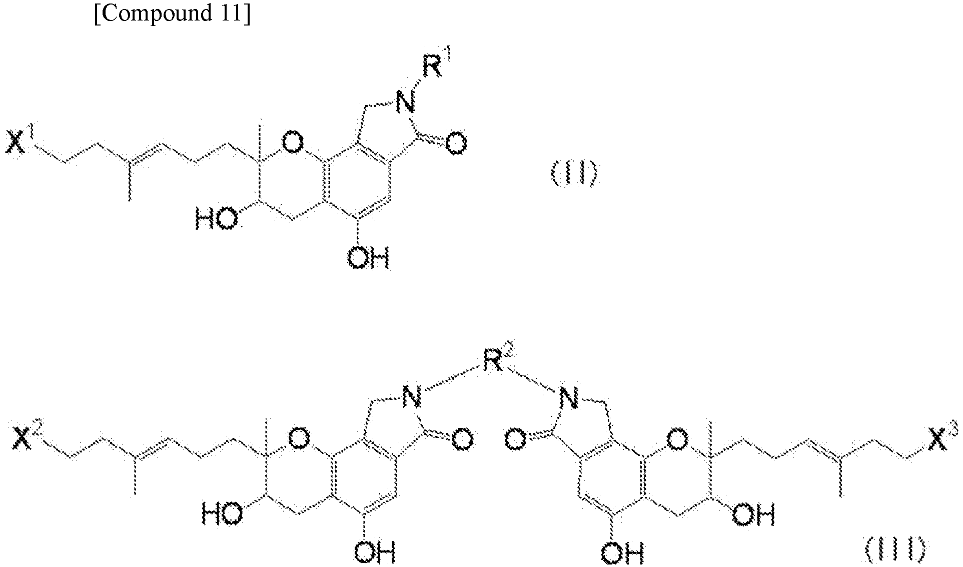 In formula (II) or formula (III), X¹, X², and X³ are independently -CHY-C(CH₃)₂Z, Y and Z are independently -H or -OH, or together form a single bond, and R¹ represents any one of (A) to (D) below.
   (A) A residue having one amino group removed from an amino compound selected from a group composed of natural amino acids, D-bodies of natural amino acids, and compounds having at least one carboxy group in a natural amino acid or D-body of a natural amino acid replaced with a hydrogen atom, a hydroxy group, or a hydroxymethyl group (however, -(CH)₂-OH is excluded),
   (B) an aromatic group having at least one selected from a group composed of a carboxy group, a hydroxy group, a sulfonic acid group, and a secondary amino group as a substituent or a part of a substituent, or an aromatic group containing a secondary amino group and that may contain a nitrogen atom,
   (C) an aromatic amino acid residue of formula (II-1) below (in the formula, R³ is a respectively independent substituent which may be present or absent, and if present, represents a hydroxy group, a carboxy group or an alkyl group having a carbon number of 1 to 5, n represents an integer of 0 or 1, m represents an integer from 0 to 5, and * represents a binding site), and 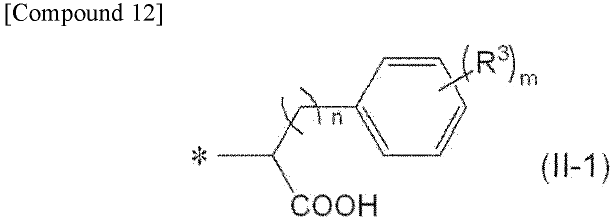
   (D) a substituent represented by -L¹-L²-R⁴ (in the formula, L¹ represents a linking group which is an alkylene group having a carbon number of 1 to 4 having a carboxy group, L² represents a linking group represented by -NH-C(=O)- or -NH-C(=S)-NH-, R⁴ represents a 9-fluorenyl alkyloxy group having an alkyloxy group having a carbon number of 1 to 3, or a polyheterocyclic group of formula (II-2) below (in formula (II-2), and * represents a binding site)). 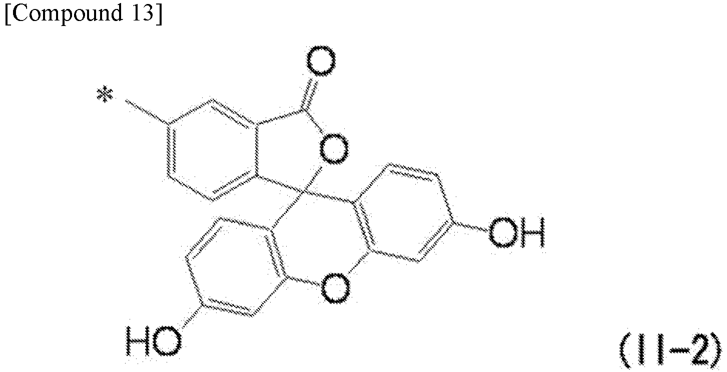 R² is a residue having two amino groups removed from an amino compound selected from a group composed of natural amino acids having 2 amino groups, D-bodies of natural amino acids having 2 amino groups, compounds having at least one carboxy group in a natural amino acid having 2 amino groups or a D-body of a natural amino acid having 2 amino groups replaced with a hydrogen atom, a hydroxy group, or a hydroxymethyl group, and compounds indicated by H₂N-CH(COOH)-(CH₂)ₙ-NH₂ (n is an integer from 0 to 9), and H₂N-CH(COOH)-(CH₂)ₘ-Sₚ-(CH₂)_{q}-CH(COOH)-NH₂ (m, p, and q are each independent integers from 0 to 9).
<12> The method according to any one of <8> to <11>, wherein the compound of the formula (I) or a pharmaceutically acceptable salt, ester, or solvate thereof is a compound selected from a group composed of SMTP-0 below, SMTP-1 below, SMTP-4 below, SMTP-5D below, SMTP-6 below, SMTP-7 below, SMTP-8 below, SMTP-11 to 14 below, SMTP-18 to 29 below, SMTP-36 below, SMTP-37 below, SMTP-42 below, SMTP-43 below, SMTP-43D below, SMTP-44 below, SMTP-44D below, SMTP-46 below, and SMTP-47 below, or a pharmaceutically acceptable salt, ester, or solvate thereof. 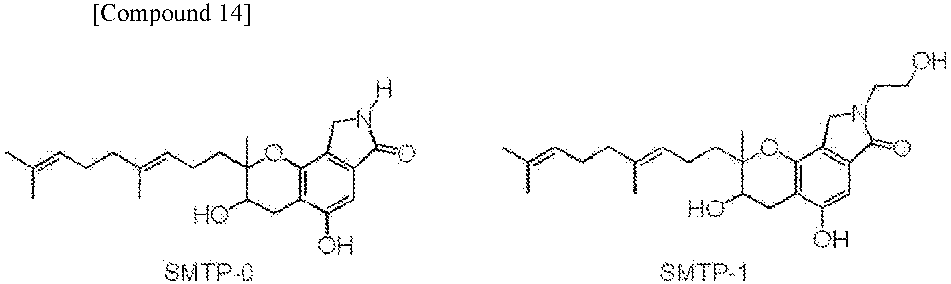 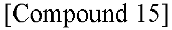 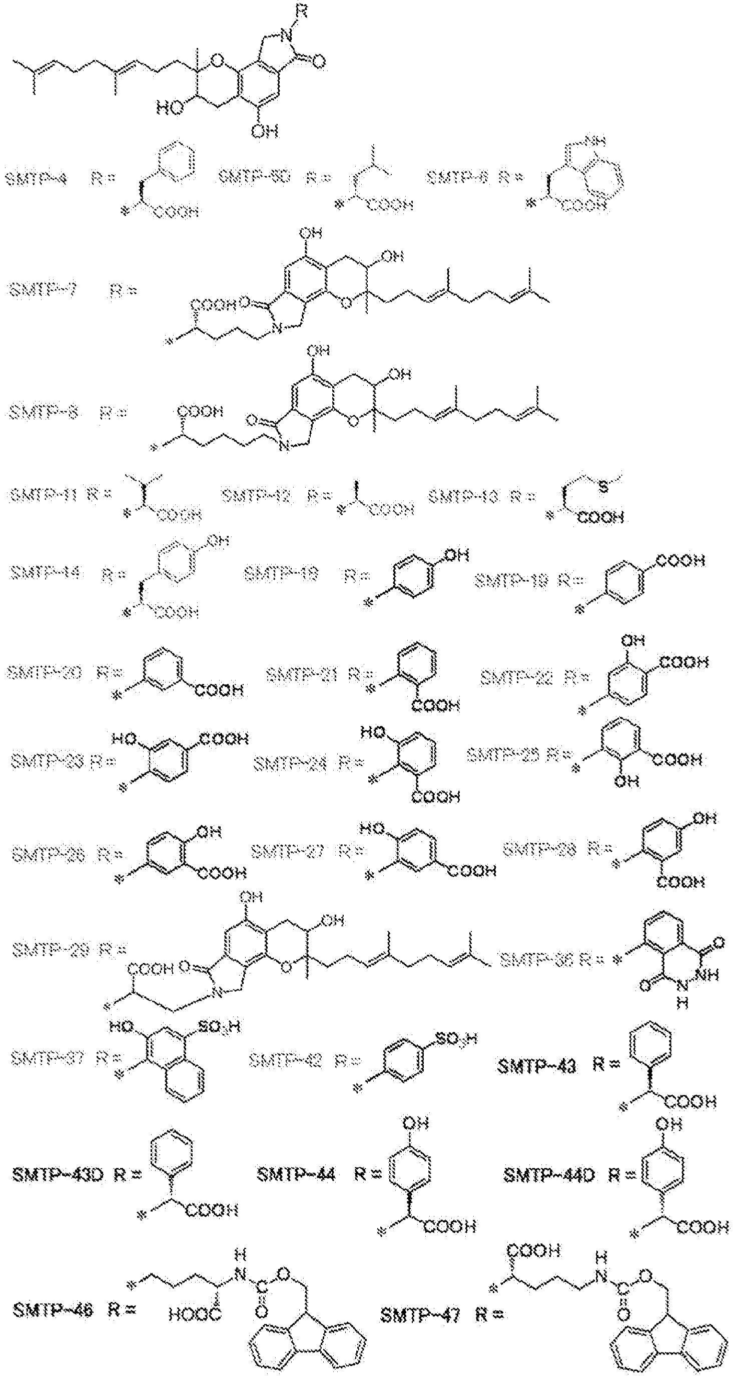 In the formula, * represents a binding site.
<13> The method according to <12>, wherein the compound of the formula (I) or a pharmaceutically acceptable salt, ester, or solvate thereof is the SMTP-7 or a pharmaceutically acceptable salt, ester, or solvate thereof.
<14> The method according to any one of <8> to <13>, wherein the cerebral hemorrhage is an intracerebral hemorrhage.
<15> The method according to any one of <8> to <14>, wherein the compound of the formula (I) or a pharmaceutically acceptable salt, ester, or solvate thereof is intracerebrally administered.
<16> A compound of the formula (I) or a pharmaceutically acceptable salt, ester, or solvate thereof for use in treatment or prevention of cerebral hemorrhage.
<17> The compound or a pharmaceutically acceptable salt, ester, or solvate thereof for use according to <16>, wherein the compound of the formula (I) or a pharmaceutically acceptable salt, ester, or solvate thereof is the compound of the formula (IA) or a pharmaceutically acceptable salt, ester, or solvate thereof.
<18> The compound or a pharmaceutically acceptable salt, ester, or solvate thereof for use according to <16> or <17>, wherein the compound of the formula (I) or a pharmaceutically acceptable salt, ester, or solvate thereof is a compound of the formula (II) or formula (III) or a pharmaceutically acceptable salt, ester, or solvate thereof.
<19> The compound or a pharmaceutically acceptable salt, ester, or solvate thereof for use according to any one of <16> to <18>, wherein the compound of the formula (I) or a pharmaceutically acceptable salt, ester, or solvate thereof is a compound selected from a group composed of the SMTP-0, the SMTP-1, the SMTP-4, the SMTP-5D, the SMTP-6, the SMTP-7, the SMTP-8, the SMTP-11 to 14, the SMTP-18 to 29, the SMTP-36, the SMTP-37, the SMTP-42, the SMTP-43, the SMTP-43D, the SMTP-44, the SMTP-44D, the SMTP-46, and the SMTP-47, or a pharmaceutically acceptable salt, ester, or solvate thereof.
<20> The compound or a pharmaceutically acceptable salt, ester, or solvate thereof for use according to <19>, wherein the compound of the formula (I) or a pharmaceutically acceptable salt, ester, or solvate thereof is the SMTP-7 or a pharmaceutically acceptable salt, ester, or solvate thereof.
<21> The compound or a pharmaceutically acceptable salt, ester, or solvate thereof for use according to any one of <16> to <20>, wherein the cerebral hemorrhage is an intracerebral hemorrhage.
<22> The compound or a pharmaceutically acceptable salt, ester, or solvate thereof for use according to any one of <16> to <21>, wherein the compound of the formula (I) or a pharmaceutically acceptable salt, ester, or solvate thereof is intracerebrally administered.
<23> A use of the compound of the formula (I) or a pharmaceutically acceptable salt, ester, or solvate thereof in production of a pharmaceutical for treatment or prevention of cerebral hemorrhage.
<24> The use according to <23>, wherein the compound of the formula (I) or a pharmaceutically acceptable salt, ester, or solvate thereof is a compound of the formula (IA) or a pharmaceutically acceptable salt, ester, or solvate thereof.
<25> The use according to <23> or <24>, wherein the compound of the formula (I) or a pharmaceutically acceptable salt, ester, or solvate thereof is a compound of the formula (II) or the formula (III) or a pharmaceutically acceptable salt, ester, or solvate thereof.
<26> The use according to any one of <23> to <26>, wherein the compound of the formula (I) or a pharmaceutically acceptable salt, ester, or solvate thereof is a compound selected from a group composed of the SMTP-0, the SMTP-1, the SMTP-4, the SMTP-5D, the SMTP-6, the SMTP-7, the SMTP-8, the SMTP-11 to 14, the SMTP-18 to 29, the SMTP-36, the SMTP-37, the SMTP-42, the SMTP-43, the SMTP-43D, the SMTP-44, the SMTP-44D, the SMTP-46, and the SMTP-47, or a pharmaceutically acceptable salt, ester, or solvate thereof.
<27> The use according to <26>, wherein the compound of the formula (I) or a pharmaceutically acceptable salt, ester, or solvate thereof is the SMTP-7 or a pharmaceutically acceptable salt, ester, or solvate thereof.
<28> The use according to any one of <23> to <27>, wherein the cerebral hemorrhage is an intracerebral hemorrhage.
<29> The use according to any one of <23> to <28>, wherein the pharmaceutical is an intracerebrally administered pharmaceutical.

### Effects of Invention

According to embodiments of the present disclosure, it is possible to provide a drug that has an excellent treatment or prevention effect of cerebral hemorrhage, as well as a new use as a pharmaceutical of a compound of formula (I) or a pharmaceutically acceptable salt, ester or solvate thereof.

### Brief Description of Drawings

[FIG. 1A] A photograph of brain sections in a study using an animal model of cerebral hemorrhage.
[FIG. 1B] A photograph of brain sections in a study using an animal model of cerebral hemorrhage.
[FIG. 1C] A photograph of brain sections in a study using an animal model of cerebral hemorrhage.
[FIG. 2] A graph showing ratios of volumes of hematoma to volumes of the whole brain in a study using an animal model of cerebral hemorrhage.
[FIG. 3A] Photographs of Evans blue dye of brain sections in a study using an animal model of cerebral hemorrhage.
[FIG. 3B] Photographs of Evans blue dye of brain sections in a study using an animal model of cerebral hemorrhage.
[FIG. 3C] Photographs of Evans blue dye of brain sections in a study using an animal model of cerebral hemorrhage.
[FIG. 3D] A photograph of Evans blue dye of brain sections in a study using an animal model of cerebral hemorrhage.
[FIG. 4] A graph showing areas of regions stained using Evans blue dye in the entire brain sample in a study using an animal model of cerebral hemorrhage.
[FIG. 5] A graph showing concentrations of Evans blue dye in the entire brain sample in a study using an animal model of cerebral hemorrhage.
[FIG. 6] MRI images of brains showing hematoma in a study using an animal model of cerebral hemorrhage.
[FIG. 7] A graph showing volume ratios of hematoma in MRI images as a percentage in a study using an animal model of cerebral hemorrhage.
[FIG. 8] A graph showing chronological changes of volume ratios (%) of hematoma in MRI images in a study using an animal model of cerebral hemorrhage.
[FIG. 9] MRI images of brains showing edema in a study using an animal model of cerebral hemorrhage.
[FIG. 10] A graph showing volume ratios of edema in MRI images as a percentage in a study using an animal model of cerebral hemorrhage.
[FIG. 11] Diagrams illustrating paths of rats in a Barnes maze test using an animal model of cerebral hemorrhage.
[FIG. 12] A graph showing a time required for rats to reach the escape box in the Barnes maze test using an animal model of cerebral hemorrhage.
[FIG. 13] A graph showing a number of holes rats incorrectly stayed at until reaching the escape box in the Barnes maze test using an animal model of cerebral hemorrhage.
[FIG. 14] A graph showing chronological changes of time required for rats to reach the escape box in the Barnes maze test using an animal model of cerebral hemorrhage.
[FIG. 15] A graph showing chronological changes of a number of holes rats incorrectly stayed at until reaching the escape box in the Barnes maze test using an animal model of cerebral hemorrhage.
[FIG. 16] A graph showing scores of each group of rats in a LAN cable walking test using an animal model of cerebral hemorrhage.
[FIG. 17] Hematoxylin and eosin (HE) stain images of brain sections in a study using an animal model of cerebral hemorrhage.
[FIG. 18] A graph showing hematoma areas in hematoxylin and eosin (HE) stain images of brain sections in a study using an animal model of cerebral hemorrhage.
[FIG. 19] Images of brain sections double stained using Luxol fast blue and Cresyl violet in a study using an animal model of cerebral hemorrhage.
[FIG. 20] A graph showing myelin density (average values in respective same sides and opposite sides of a collagenase injection of white matter) in images of brain sections double stained using Luxol fast blue and Cresyl violet in a study using an animal model of cerebral hemorrhage.
[FIG. 21] A graph showing myelin density (values of each specimen in the center of white matter) in images of brain sections double stained using Luxol fast blue and Cresyl violet in a study using an animal model of cerebral hemorrhage.
[FIG. 22] Antibody-stained images of brain sections using antibodies against cleaved caspase-3 in a study using an animal model of cerebral hemorrhage.
[FIG. 23] Antibody-stained images of brain sections using antibodies against cleaved caspase-3 in a study using an animal model of cerebral hemorrhage.
[FIG. 24] A graph showing a number of immunopositive cells per 100 µm² in antibody-stained images of brain sections using antibodies against cleaved caspase-3 in a study using an animal model of cerebral hemorrhage.
[FIG. 25] Antibody-stained images of brain sections using anti-GFAP antibodies in a study using an animal model of cerebral hemorrhage.
[FIG. 26] Antibody-stained images of brain sections using anti-GFAP antibodies in a study using an animal model of cerebral hemorrhage.
[FIG. 27] A graph showing a number of immunopositive cells per 100 µm² in antibody-stained images of brain sections using anti-GFAP antibodies in a study using an animal model of cerebral hemorrhage.
[FIG. 28] Antibody-stained images of brain sections using anti-Iba-1 antibodies in a study using an animal model of cerebral hemorrhage.
[FIG. 29] Antibody-stained images of brain sections using anti-Iba-1 antibodies in a study using an animal model of cerebral hemorrhage.
[FIG. 30] A graph showing a number of immunopositive cells per 100 µm² in antibody-stained images of brain sections using anti-Iba-1 antibodies in a study using an animal model of cerebral hemorrhage.

### Description of Embodiments

The content of the present disclosure will be described in detail below. While the description of configuration requirements described below may be based on representative embodiments of the present disclosure, the present disclosure is not limited to such embodiments.

In numerical ranges described in steps in the present disclosure, the upper or lower limits described for one numerical range may be replaced by the upper or lower limits of another numerical range described in steps. Moreover, in numerical ranges described in the present disclosure, the upper or lower limits of the numerical range may be replaced by values shown in the examples.

Furthermore, in the present disclosure, the amount of each component in a composition such as a drug, when a plurality of substances applicable to each component in the composition are present, means the total amount of the applicable plurality of substances present in the composition, unless otherwise specified.

Moreover, in the notation of groups (atomic groups) in the present specification, those not marked as substituted and unsubstituted include those without substituents as well as those with substituents.

Moreover, the term "process" in the present specification is included as a present term not only for independent processes, but also for processes that cannot be clearly distinguished from other processes, so long as the desired object of the process is achieved.

Moreover, in the present disclosure, "mass%" and "weight%" are synonymous, and "mass part" and "weight part" are synonymous.

Furthermore, in the present disclosure, a combination of two or more preferable aspects is the more preferred aspect.

The present disclosure will be described in detail below.

### (Drug)

The drug according to the present disclosure is a drug used for the treatment or prevention of cerebral hemorrhage, containing as an active ingredient a compound of formula (I) above or a pharmaceutically acceptable salt, ester, or solvate thereof. Here, a pharmaceutically acceptable salt, ester, or solvate of a compound of formula (I) above may be obtained from a compound of formula (I) by an ordinary method. For this reason, in the following description, unless specified otherwise, a description of each instance of a salt, ester, or solvate is omitted, and the description of a "compound of formula (I)" shall be applied in the same manner as "pharmaceutically acceptable salt, ester, or solvate of a compound of formula (I)".

### <Compound of Formula (I)>

The drug of the present disclosure is a compound of formula (I) or a pharmaceutically acceptable salt, ester, or solvate thereof.

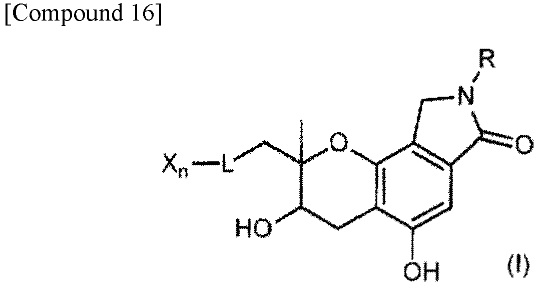

In formula (I), L represents an aliphatic hydrocarbon group having a carbon number of 4 to 10, X represents a hydroxy group or carboxy group, n represents an integer from 0 to 2, and R represents a hydrogen atom or a substituent having a molecular weight of 1,000 or less.

The aliphatic hydrocarbon group having a carbon number of 4 to 10 indicated by L may be straight-chain, branched-chain, or cyclic. It may also contain unsaturated bonds. Among these, an aliphatic hydrocarbon group that may contain straight-chain or branched-chain unsaturated bonds is preferable. L is an n+1 valency group.

In formula (I), the group represented by -L-Xₙ is preferably a group selected from a group composed of the following formula (V) and formulas (Y1) to (Y4). In formula (V) and formulas (Y1) to (Y4), * represents a binding site to a carbon atom adjacent to the oxygen-containing ring in formula (I) (the carbon atom to which L is bound).

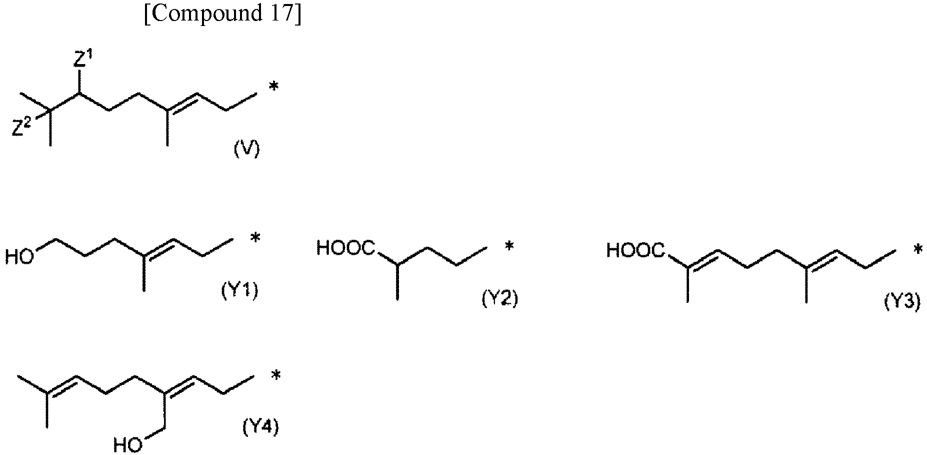

In formula (V), Z¹ and Z² are an independent hydrogen atom or hydroxy group, or together form a single bond.

In R of formula (I), as a substituent having a molecular weight of 1,000 or less, from the perspective of reducing hematoma and edema described later, a substituent having a molecular weight of 900 or less is preferable, a substituent having a molecular weight of 800 or less is more preferable, and a substituent having a molecular weight of 700 or less is further preferable.

An α-amino acid is an example of R in formula (I) (in this case, the nitrogen atom that binds to R is an α-amino group of the α-amino acid). The α-amino acid is not particularly limited and may be a natural amino acid or non-natural amino acid. It may also be an amino acid derivative in which a substituent is introduced into a natural amino acid. Furthermore, if the α-amino acid has two or more amino groups, any of the amino groups may be removed. Moreover, amino sugars, heterocyclic groups, and the like are examples of R.

Among these, the α-amino acid is preferably a natural amino acid, a D-body of a natural amino acid, or phenylalanine or phenylglycine which may have at least one type of substituent selected from a group composed of a hydroxy group, a carboxy group, and an alkyl group having a carbon number of 1 to 5, and is more preferably a natural amino acid, a D-body of a natural amino acid, or phenylglycine which may have at least one type of substituent selected from a group composed of a hydroxy group, a carboxy group, and an alkyl group having a carbon number of 1 to 5. Here, "D-body of a natural amino acid" means the D-type optical isomer of a natural amino acid (basically, L-type).

The natural amino acid is not particularly limited so long as it is an amino acid that can occur naturally. Examples include glycine, alanine, threonine, valine, isoleucine, tyrosine, cysteine, cystine, methionine, histidine, aspartic acid, glutamic acid, asparagine, glutamine, arginine, lysine, hydroxylysine, ornithine, citrulline, homocysteine, 3,4-dihydroxyphenylalanine, homocysteine, diaminopimelic acid, diaminopropionic acid, serine, leucine, phenylalanine, tryptophan, and the like.

Examples of a substituent in an amino acid derivative in which a substituent is introduced into a natural amino acid include a nitro group, a hydroxy group, an arylalkyl group having a carbon number of 7 to 16, a ureido group, a thioureido group, a carboxy group, a group constituted having one hydrogen atom removed from fluorescamine, and the like. A substituent in the amino acid derivative may have a further substituent when possible. The substituent having a substituent is the same as the substituent in the amino acid derivative.

The amino sugar in R of formula (I) is not particularly limited so long as it is a sugar derivative having at least one amino group. Specific examples may include glucosamine, galactosamine, mannosamine, neuraminic acid, and the like.

The heterocyclic group in R of formula (I) is not particularly limited so long as it is a cyclic group containing a heteroatom, and may be either an aliphatic heterocyclic group or an aromatic heterocyclic group. Moreover, a nitrogen atom, an oxygen atom, a sulfur atom, and the like are examples of a heteroatom.

Among these, a nitrogen-containing heterocyclic group containing a nitrogen atom as a heteroatom is preferable, a heterocyclic group constituted having one hydrogen atom removed from a heterocyclic compound selected from a group composed of purine, pyridine, pyridazine, pyrrole, imidazole, pyrazole, and pyrazolone is more preferable, and a heterocyclic group constituted having one hydrogen atom removed from a heterocyclic compound selected from a group composed of purine, pyridine, and pyrazolone is further preferable. Note that a position at which the hydrogen atom is removed from the heterocyclic compound is not particularly limited. Among these, removing from the carbon atom on the heterocyclic compound is preferable.

The heterocyclic group in R may have a substituent. Examples of the substituent in the heterocyclic group include an alkyl group having a carbon number of 1 to 5, an aryl group having a carbon number of 14 or less, a carboxy group, a carbamoyl group, a sulfonic acid group, and the like. Among these, at least one type selected from a group composed of a phenyl group and a carbamoyl group is preferable.

A number of substituents in the heterocyclic group is not particularly limited, but three or fewer is preferable.

R in formula (I) may be an alkyl group, for example, an alkyl group having a carbon number of 2 to 8. The alkyl group having a carbon number of 2 to 8 may be straight-chain, branched-chain, or cyclic. Among these, straight-chain or branched-chain is preferable, and straight-chain is more preferable. Moreover, the carbon number is preferably 2 to 6. Note that the carbon number of the substituent on the alkyl group is not included in the carbon number of the alkyl group.

The alkyl group in R may have a substituent. Examples of the substituent in the alkyl group include an alkyl group having a carbon number of 1 to 5, an aryl group having a carbon number of 14 or less, an arylalkyl group having a carbon number of 16 or less, a hydroxy group, a carboxy group, a carbamoyl group, a sulfonic acid group, an amino group, a carbamoyloxy group, a ureido group, a thioureido group, an alkyl sulfide group, an alkyl disulfide group, a group constituted having R removed from a compound of formula (I), a group constituted having one hydrogen atom removed from fluorescamine, and the like. Among these, at least one type selected from a group composed of a hydroxy group, a carboxy group, an amino group, a carbamoyloxy group, an arylalkyl group having a carbon number of 7 to 14, a thioureido group, a group constituted having R removed from a compound of formula (I), and a group constituted having one hydrogen atom removed from fluorescamine is preferable.

A number of substituents in the alkyl group is not particularly limited, but three or fewer is preferable.

Moreover, a substituent in the alkyl group may have a further substituent when possible. The substituent having a substituent is the same as the substituent in the alkyl acid.

R in formula (I) may be an aryl group. The aryl group is preferably an aryl group having a carbon number of 6 to 14, more preferably an aryl group having a carbon number of 6 to 10, and further preferably a phenyl group.

The aryl group in R may have a substituent. Examples of the substituent in the aryl group include an alkyl group having a carbon number of 1 to 5, an aryl group having a carbon number of 14 or less, a hydroxy group, a carboxy group, a sulfonic acid group, a carbamoyl group, an aryl carbonyl group, and the like. Among these, at least one type selected from a group composed of a hydroxy group, a carboxy group, a sulfonic acid group, a carbamoyl group, and an aryl carbonyl group is preferable.

A number of substituents in the aryl group is not particularly limited, but three or fewer is preferable.

Moreover, a substituent in the aryl group may have a further substituent when possible. The substituent having a substituent is the same as the substituent in the aryl group. Furthermore, substituents in the aryl group may bind together to form a cyclic structure when possible.

### <Production Method of a Compound of Formula (I)>

A compound of formula (I) used in the present disclosure may be obtained by chemical synthesis or obtained by purification from cultures of filamentous fungi, for example, *stachybotrys microspora* (*stachybotrys microspora*). Methods for obtaining a compound of formula (I) by purification from cultures of filamentous fungi include a method involving purification of the target compound from a culture obtained when a predetermined added organic amino compound is added to a culture medium of *stachybotrys microspora.* These methods are taught in, for example, JP 2004-224737 A, JP 2004-224738 A, and WO 2007/111203.

A compound of formula (I) used in the present disclosure may be an enantiomer, a diastereomer, or a mixture of enantiomers or of diastereomers. An enantiomer, a diastereomer, or mixture of enantiomers or of diastereomers may be obtained by chemical synthesis or obtained by purification from cultures of filamentous fungi. In the case of obtaining by purification from cultures of filamentous fungi, by adding a D-body or L-body of an added organic amino compound to culture the filamentous fungi, isomers of the D-body or L-body can be obtained.

### <Compound of Formula (IA)>

The compound of the formula (I) is preferably a compound of formula (IA) below.

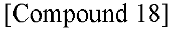

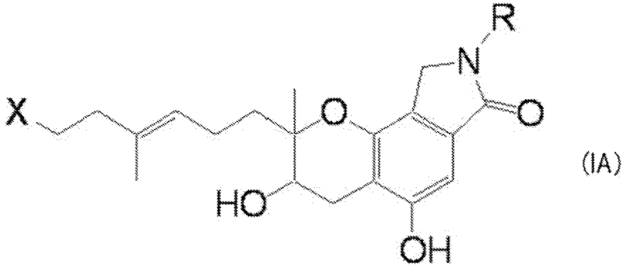

In formula (IA), X is -CHY-C(CH₃)₂Z, and Y and Z are independently -H or -OH, or together form a single bond. R represents a hydrogen atom or a substituent having a molecular weight of 1,000 or less.

R in formula (IA) is the same as R in formula (I), and is also the same as a preferable aspect.

### <Compound of Formula (II)>

One specific example of a compound of formula (I) used in the present disclosure is a compound of formula (II) below.

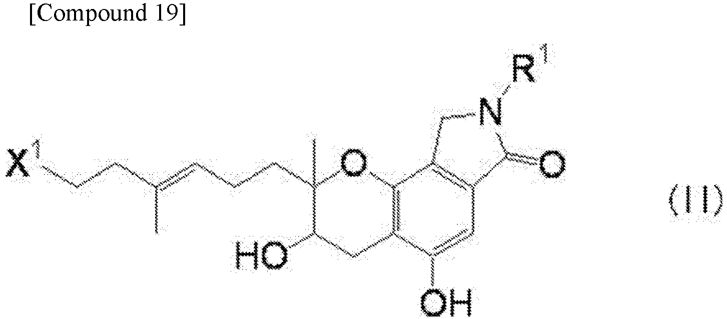

In formula (II), X¹ is -CHY-C(CH₃)₂Z, Y and Z are independently -H or -OH, or together form a single bond, and R¹ represents any one of (A) to (D) below.
(A) A residue having one amino group removed from an amino compound selected from a group composed of natural amino acids, D-bodies of natural amino acids, and compounds having at least one carboxy group in a natural amino acid or D-body of a natural amino acid replaced with a hydrogen atom, a hydroxy group, or a hydroxymethyl group (however, -(CH)₂-OH is excluded),
(B) an aromatic group having at least one selected from a group composed of a carboxy group, a hydroxy group, a sulfonic acid group, and a secondary amino group as a substituent or a part of a substituent, or an aromatic group containing a secondary amino group and that may contain a nitrogen atom,
(C) an aromatic amino acid residue of formula (II-1) below (in the formula, R³ is a respectively independent substituent which may be present or absent, and if present, represents a hydroxy group, a carboxy group or an alkyl group having a carbon number of 1 to 5, n represents an integer of 0 or 1, m represents an integer from 0 to 5, and * represents a binding site to a nitrogen atom (nitrogen atom to which R₁ is bound) in a nitrogen-containing 5-membered ring in formula (II)), and 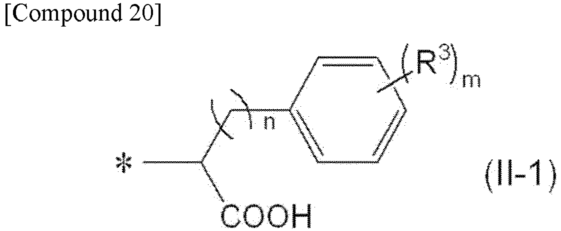
(D) a substituent represented by -L¹-L²-R⁴ (in the formula, L¹ represents a linking group which is an alkylene group having a carbon number of 1 to 4 having a carboxy group, L² represents a linking group represented by -NH-C(=O)- or -NH-C(=S)-NH-, R⁴ represents a 9-fluorenyl alkyloxy group having an alkyloxy group having a carbon number of 1 to 3, or a polyheterocyclic group of formula (II-2) below). In formula (II-2), * represents the binding site to L2.

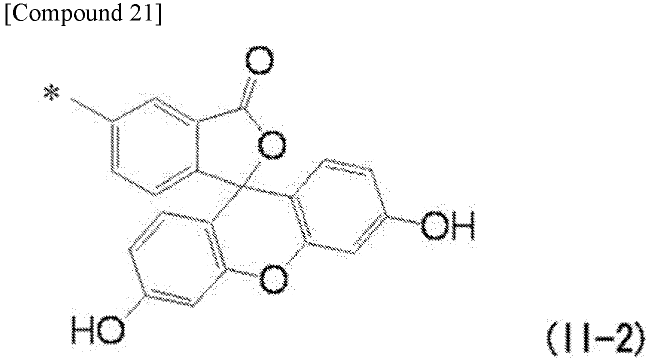

In formula (II), a compound will be described when R¹ is (A) above.
(A) above is a residue having one amino group removed from an amino compound selected from a group composed of natural amino acids, D-bodies of natural amino acids, and compounds having at least one carboxy group in a natural amino acid or D-body of a natural amino acid replaced with a hydrogen atom, a hydroxy group, or a hydroxymethyl group (however, -(CH)₂-OH is excluded).

The natural amino acid is not particularly limited so long as it is an amino acid that can naturally exist, for example, an α-amino acid, a β-amino acid, a γ-amino acid, a δ-amino acid, or the like. Such amino acids may be obtained from natural products or may be obtained artificially by methods such as organic synthesis.

Natural amino acids include substances such as the following. Examples of α-amino acids include glycine, alanine, threonine, valine, isoleucine, tyrosine, cysteine, cystine, methionine, histidine, aspartic acid, glutamic acid, asparagine, glutamine, arginine, lysine, hydroxylysine, ornithine, citrulline, homocysteine, 3,4-dihydroxyphenylalanine, homocysteine, diaminopimelic acid, diaminopropionic acid, serine, leucine, phenylalanine, tryptophan, and the like, examples of β-amino acids include β-alanine and the like, examples of γ-amino acids include γ-aminobutyric acid, carnitine, and the like, and examples of δ-amino acids include 5-aminolevulinic acid, 5-aminovaleric acid, and the like.

Compounds in which at least one carboxy group is replaced by a hydrogen atom, hydroxy group or hydroxymethyl group in the above natural amino acid or D-body of a natural amino acid include, for example, amino alcohols and amines. Such amino alcohols include, for example, 2-aminoethanol, or the like.

Specific examples of compounds when R¹ in formula (II) is (A) above include the compounds shown in table 1 below. Note that "added organic amino compound" in the table indicates the added organic amino compound added to the *stachybotrys microspora* culture medium to obtain the target compound (the same applies hereinafter). In the table, * represents a binding site to a nitrogen atom (nitrogen atom to which R is bound) in a nitrogen-containing 5-membered ring in the structural formula described at the top of the table (the same applies hereinafter).

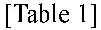

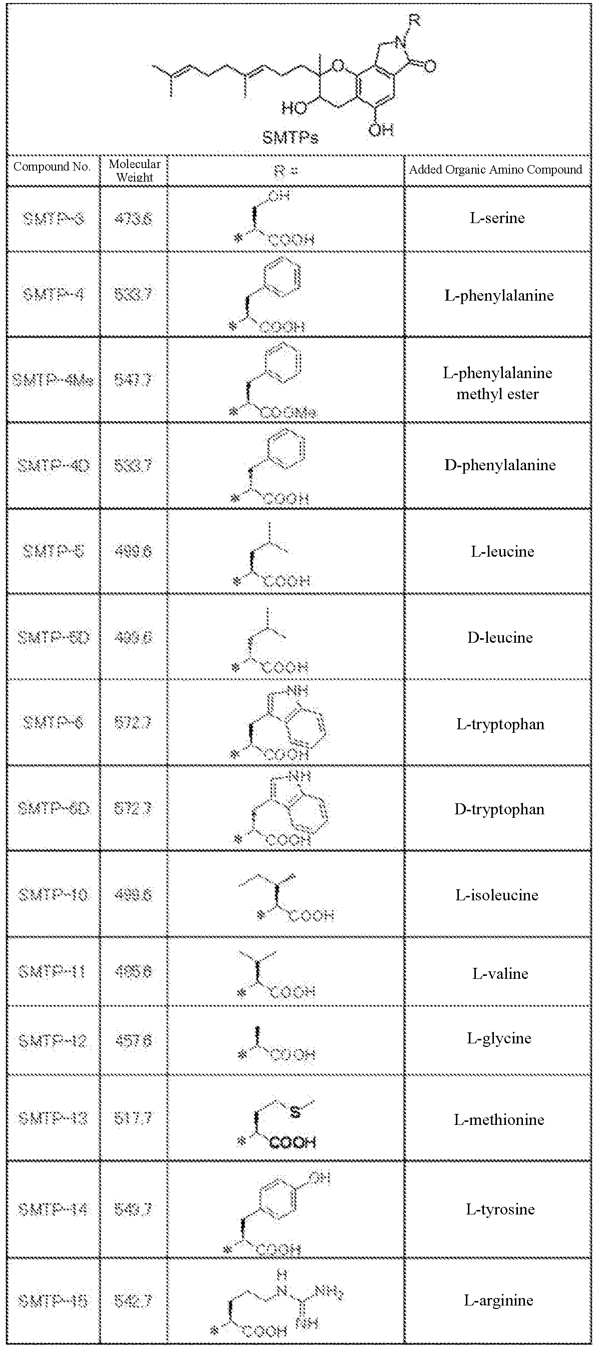

The compounds shown in table 1 above may be ideally used as compounds of formula (I) used in the present disclosure.

In formula (II), a compound will be described when R¹ is (B) above.

(B) above is an aromatic group having at least one selected from a group composed of a carboxy group, a hydroxy group, a sulfonic acid group, and a secondary amino group as a substituent or a part of a substituent, or an aromatic group containing a secondary amino group and that may contain a nitrogen atom.

The aromatic groups include, for example, groups represented by the following structural formulas. In each structural formula, * represents a binding site to a nitrogen atom (nitrogen atom to which R¹ is bound) in a nitrogen-containing 5-membered ring in formula (II).

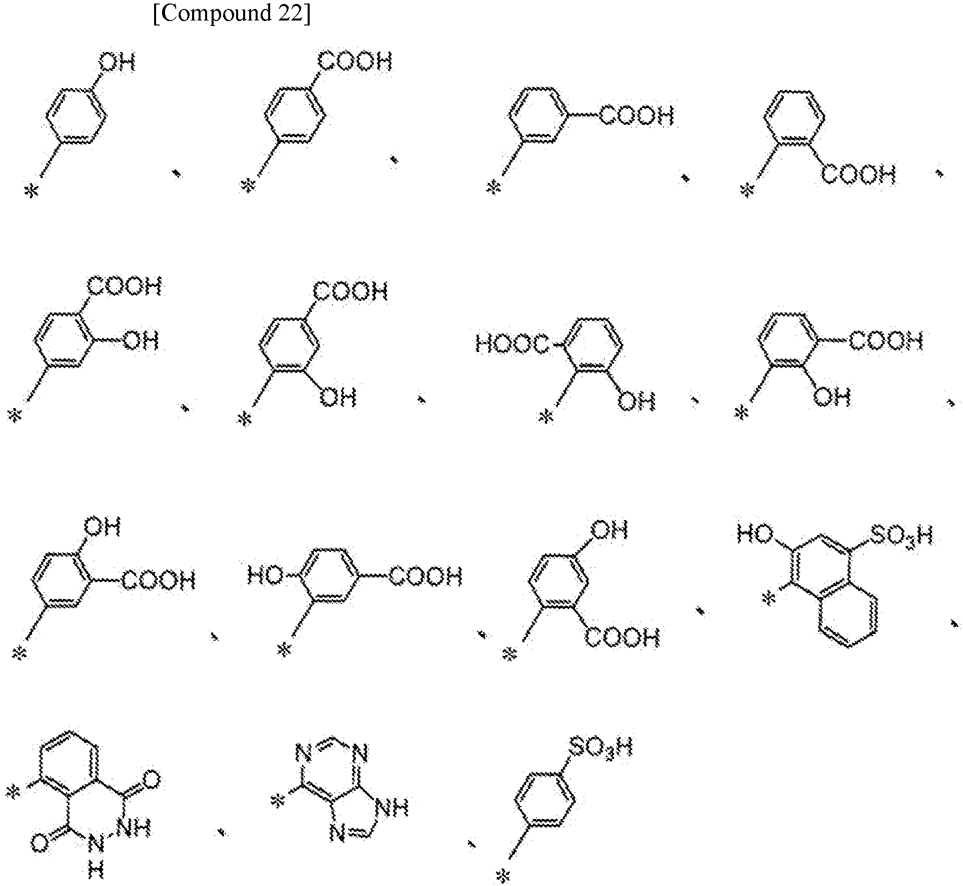

Specific examples of compounds when R¹ in formula (II) is (B) above include the compounds shown in table 2 below. In the table, * represents a binding site to a nitrogen atom (nitrogen atom to which R is bound) in a nitrogen-containing 5-membered ring in the structural formula described at the top of the table.

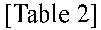

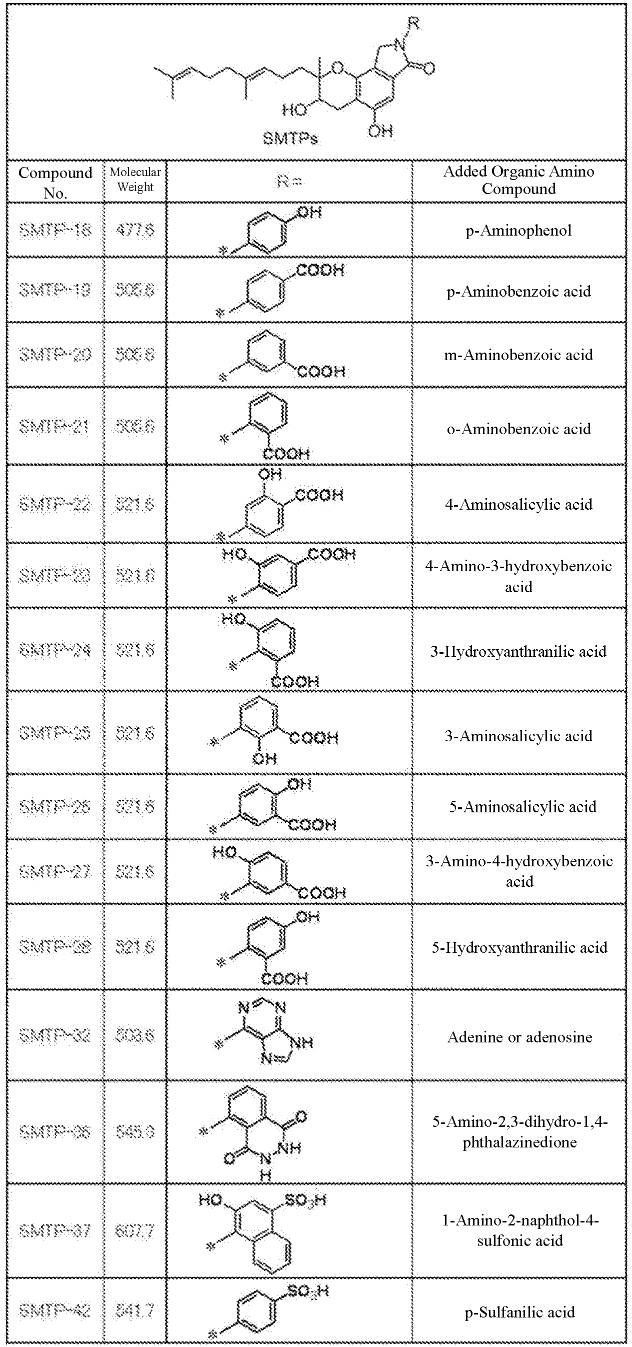

The compounds shown in table 2 above may be ideally used as compounds of formula (I) used in the present disclosure.

In formula (II), a compound will be described when R¹ is (C) above.
(C) above is an aromatic amino acid residue of formula (II-1) below (in the formula, R³ is a substituent which may be present or absent, and if present, represents at least one substituent selected from a group composed of a hydroxy group, a carboxy group, or an alkyl group having a carbon number of 1 to 5, n represents an integer of 0 or 1, m represents an integer from 0 to 5, and * represents a binding site. The alkyl group may further have a substituent, and examples of the substituent include a hydroxy group, an alkenyl group, an amino group, a carboxyl group, a sulfhydryl group, and the like).

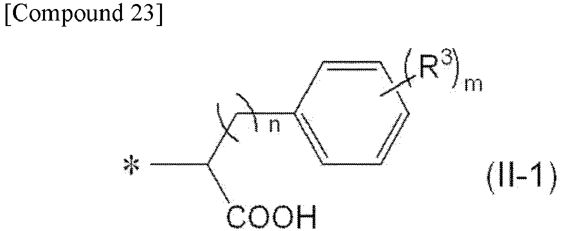

Aromatic amino acid residues of the formula (II-1) include, for example, groups represented by the following structural formulas. * represents a binding site to a nitrogen atom (nitrogen atom to which R₁ is bound) in a nitrogen-containing 5-membered ring in formula (II).

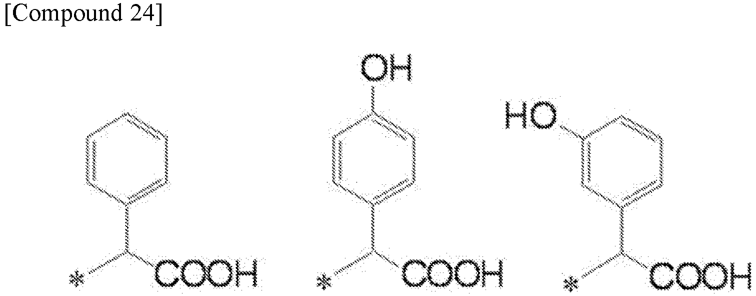

Specific examples of compounds when R¹ in formula (II) is (C) above include the compounds shown in table 3 below. In the table, * represents a binding site to a nitrogen atom (nitrogen atom to which R is bound) in a nitrogen-containing 5-membered ring in the structural formula described at the top of the table

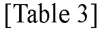

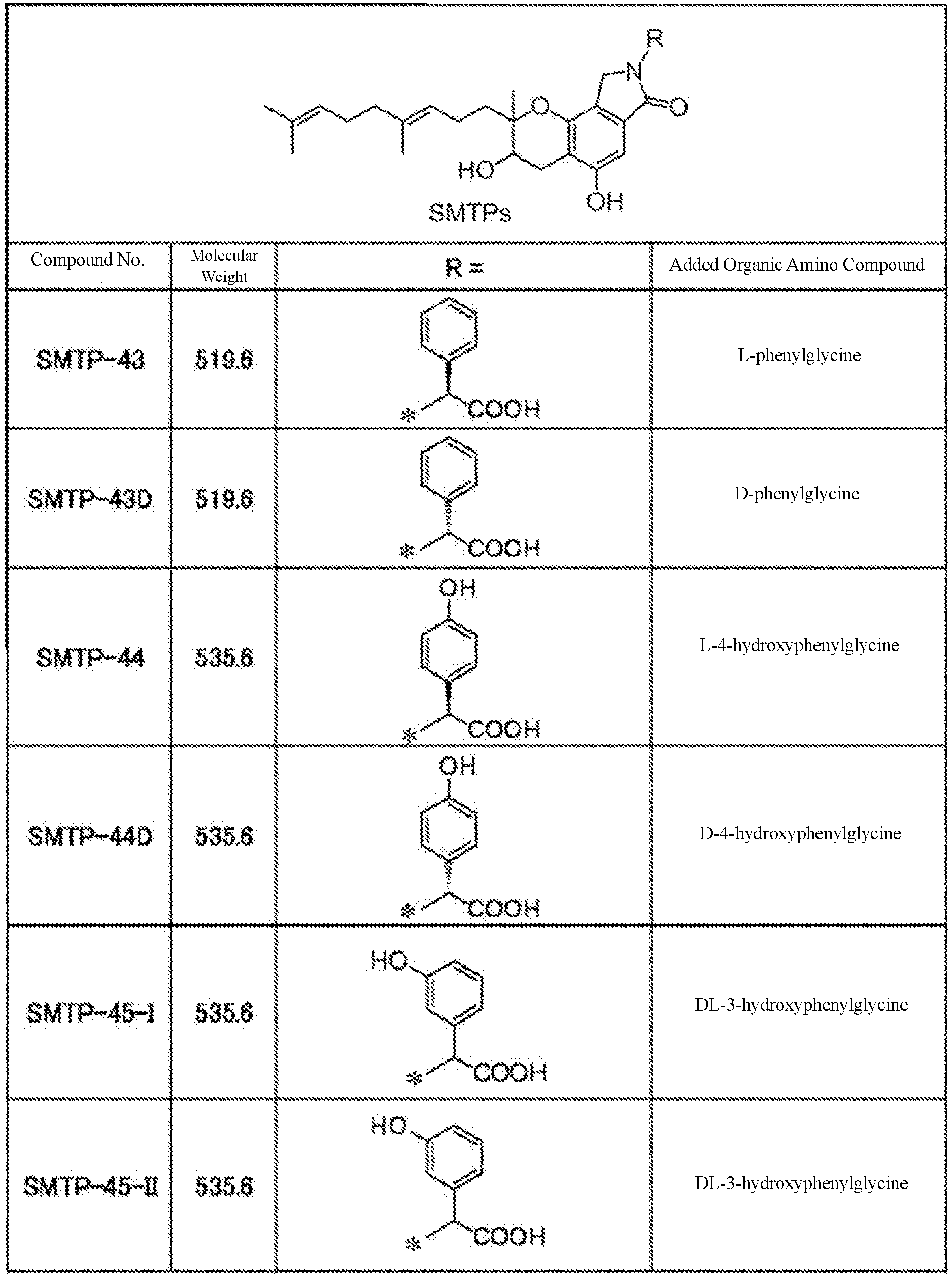

The compounds shown in table 3 above may be ideally used as compounds of formula (I) used in the present disclosure.

In formula (II), a compound will be described when R¹ is (D) above.
(D) above is a substituent represented by -L¹-L²-R⁴ (in the formula, L¹ represents a linking group which is an alkylene group having a carbon number of 1 to 4 having a carboxy group, L² represents a linking group represented by -NH-C(=O)- or -NH-C(=S)-NH-, and R⁴ represents a 9-fluorenyl alkyloxy group having an alkyloxy group having a carbon number of 1 to 3, or a polyheterocyclic group of formula (II-2) below). In the formula below, * represents a binding site to a nitrogen atom (nitrogen atom to which R₁ is bound) in a nitrogen-containing 5-membered ring in formula (II).

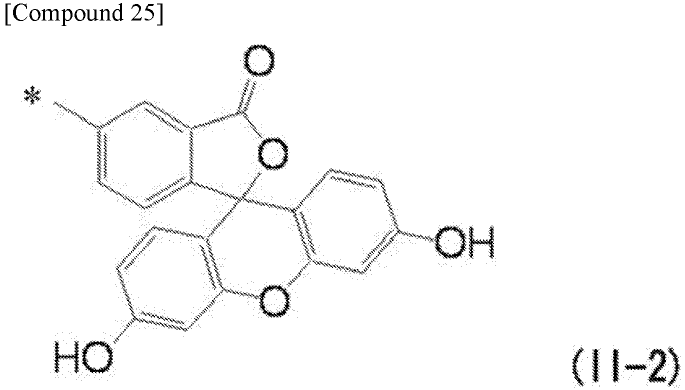

Specific examples of compounds when R¹ in formula (II) is (D) above include the compounds shown in table 4 below. In the table, * represents a binding site to a nitrogen atom (nitrogen atom to which R is bound) in a nitrogen-containing 5-membered ring in the structural formula described at the top of the table

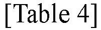

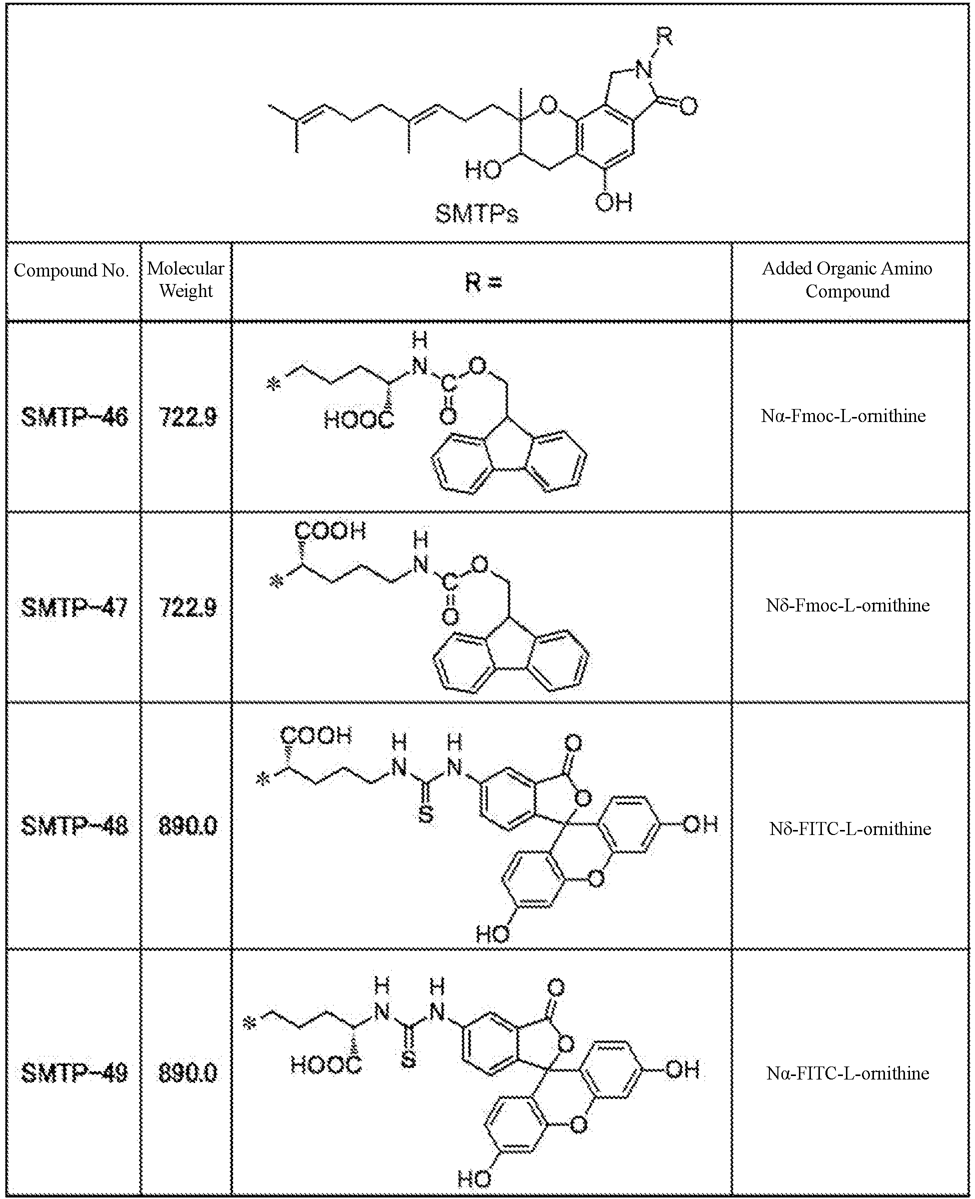

The compounds shown in table 4 above may be ideally used as compounds of formula (I) used in the present disclosure.

### <Compound of Formula (III)>

One specific example of a compound of formula (I) used in the present disclosure is a compound of formula (III) below.

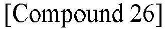

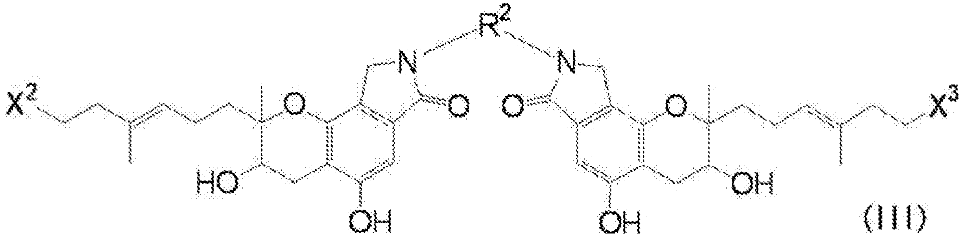

In formula (III), X² and X³ are independently -CHY-C(CH₃)₂Z, and Y and Z are independently -H or -OH, or together form a single bond. R² represents a residue having two amino groups removed from an amino compound selected from a group composed of natural amino acids having 2 amino groups, D-bodies of natural amino acids having 2 amino groups, compounds having at least one carboxy group in a natural amino acid having 2 amino groups or a D-body of a natural amino acid having 2 amino groups replaced with a hydrogen atom, a hydroxy group, or a hydroxymethyl group, and compounds indicated by H₂N-CH(COOH)-(CH₂)ₙ-NH₂ (n is an integer from 0 to 9), and H₂N-CH(COOH)-(CH₂)ₘ-Sₚ-(CH₂)_{q}-CH(COOH)-NH₂ (m, p, and q are each independent integers from 0 to 9).

The n represents an integer from 0 to 9, preferably from 0 to 6, more preferably from 1 to 5, and further preferably from 1 to 4.

The m represents an integer from 0 to 9, preferably from 0 to 4, more preferably from 1 to 3, and further preferably 1 or 2.

The p represents an integer from 0 to 9, preferably from 0 to 4, more preferably from 1 to 3, and further preferably 1 or 2.

The q represents an integer from 0 to 9, preferably from 0 to 4, more preferably from 1 to 3, and further preferably 1 or 2.

When the p is 0, m+q is preferably an integer from 0 to 9, more preferably from 0 to 6, further preferably from 1 to 5, and particularly preferably from 1 to 4.

Natural amino acids having two amino groups include, for example, hydroxylysine, citrulline, cystine, homocystine, diaminopimelic acid, diaminopropionic acid, lysine, and ornithine as α-amino acids.

Compounds in which at least one carboxy group is replaced by a hydrogen atom, a hydroxy group or a hydroxymethyl group in a natural amino acid having two amino groups and a D-body of a natural amino acid having two amino groups include H₂N-(CH₂)ₖ-NH₂ (k is an integer from 1 to 10, preferably from 1 to 6, and more preferably from 1 to 4).

Specific examples of compounds in formula (III) include compounds shown in table 5 below. In the table, * represents a binding site to a nitrogen atom (nitrogen atom to which R is bound) in a nitrogen-containing 5-membered ring in the structural formula described at the top of the table

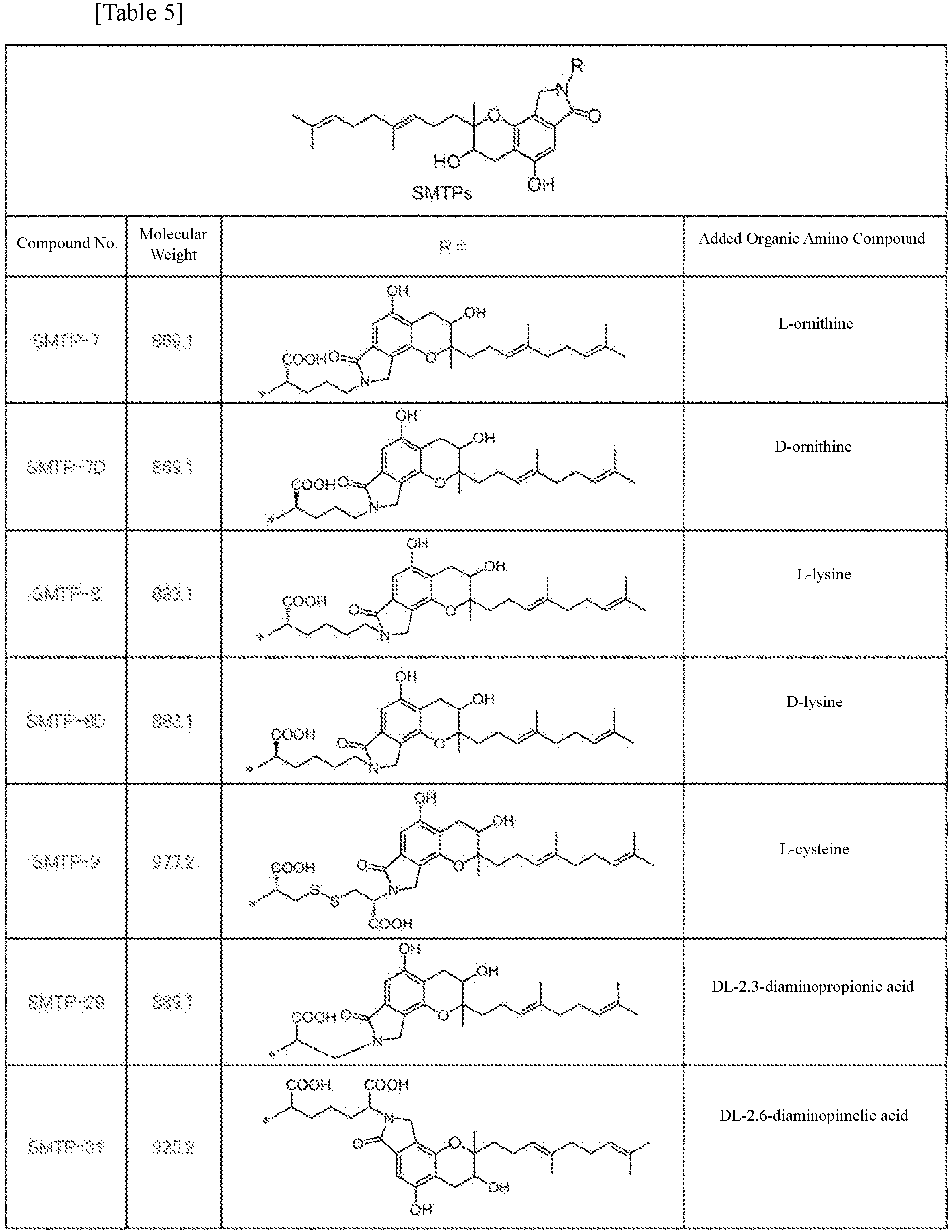

The compounds shown in table 5 above may be ideally used as compounds of formula (I) used in the present disclosure.

In addition to the compounds of the formula (II) or (III), specific examples of compounds of the formula (I) include compounds shown in tables 6 through 8 below. In table 7, * represents a binding site to a nitrogen atom (nitrogen atom to which Rb or Rc is bound) in a nitrogen-containing 5-membered ring in the structural formula ((Ib) or (Ic)) described at the top of the table. In table 8, * represents a binding site to a nitrogen atom (nitrogen atom to which Rd or Re is bound) in a nitrogen-containing 5-membered ring in the structural formula ((Id) or (Ie)) described at the top of the table.

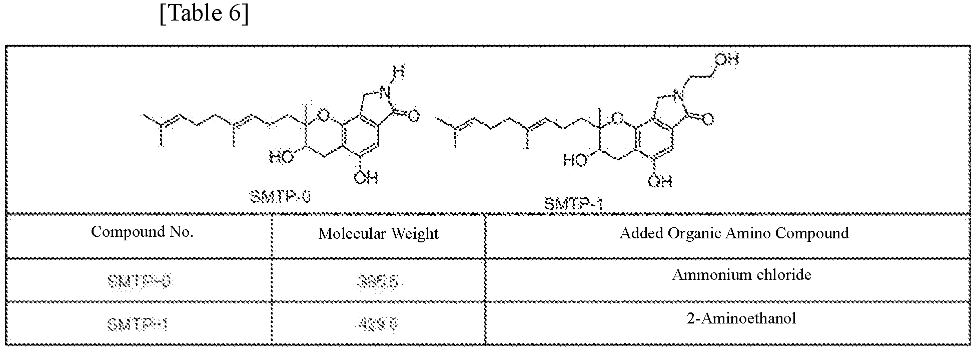

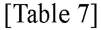

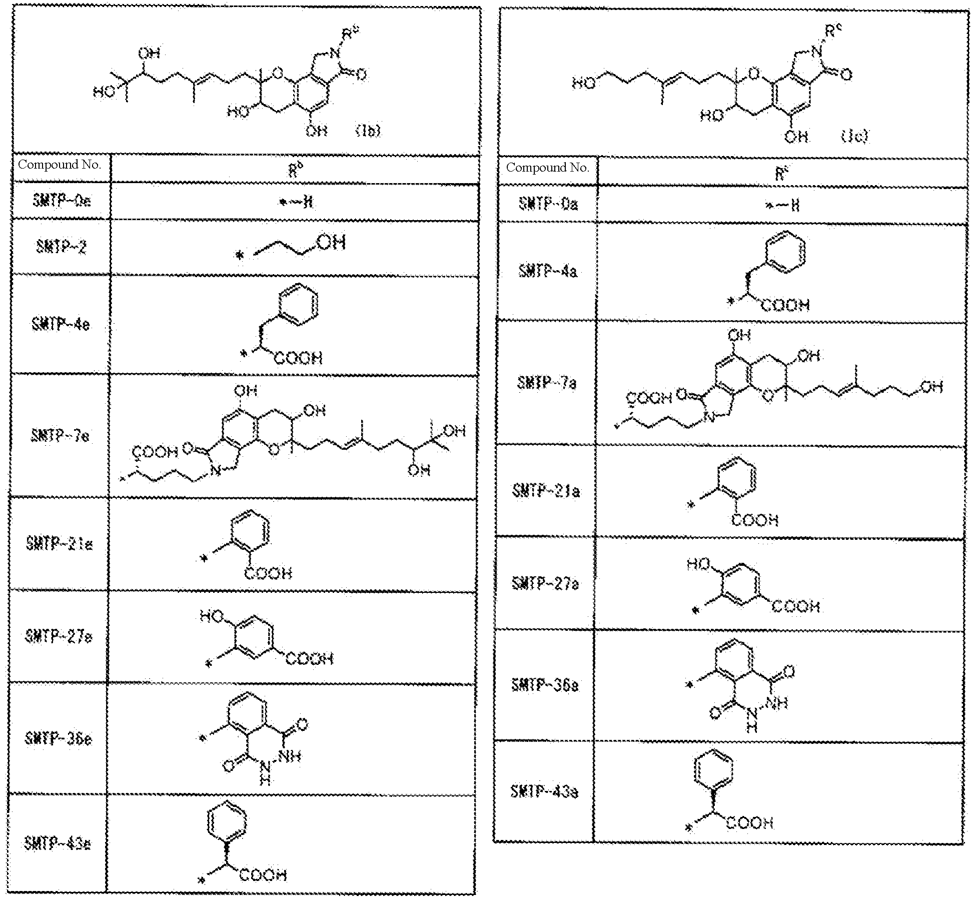

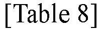

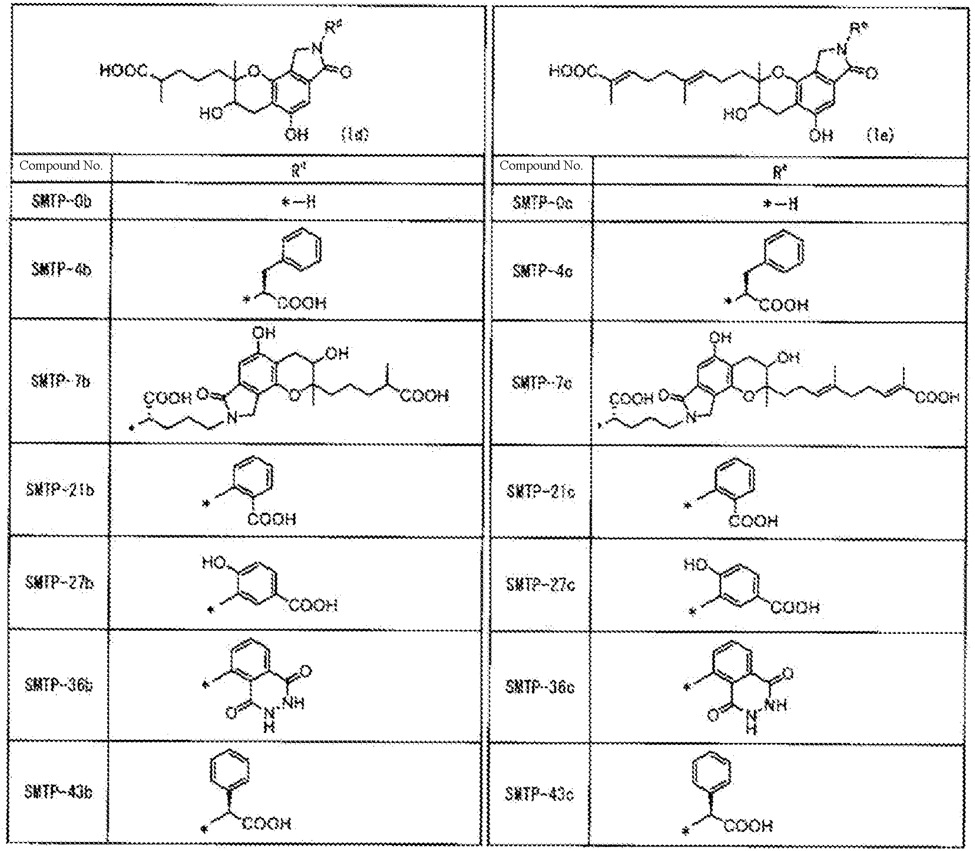

The compounds shown in table 6 to table 8 above may be ideally used as compounds of formula (I) included in a drug.

Among the above compounds, as a compound of formula (I), SMTP-0, SMTP-1, SMTP-4, SMTP-5D, SMTP-6, SMTP 7, SMTP-8, SMTP-11 to 14, SMTP-18 to 29, SMTP-36, SMTP-37, SMTP-42, SMTP-43, SMTP-43D, SMTP-44, SMTP-44D, SMTP-46 and SMTP-47 are preferable, SMTP-7, SMTP-19, SMTP-22, SMTP-43, and SMTP-44D are more preferable, and SMTP-7 is further preferable. One of these compounds may be used alone or two or more may be used in any combination.

Compounds of formula (I) used in the present disclosure may be any among a free form, a form of a pharmaceutically acceptable salt or ester, or a form of a solvate. Inorganic or organic acids such as hydrochloric acid, hydrobromic acid, sulfuric acid, nitric acid, phosphoric acid, or citric acid, formic acid, fumaric acid, malic acid, acetic acid, succinic acid, tartaric acid, methanesulfonic acid, and p-toluenesulfonic acid are ideal in the form of pharmaceutically acceptable salts of compounds of formula (I) used in the present disclosure. Moreover, compounds containing alkali metals or alkali earth metals, such as sodium, potassium, calcium, magnesium, or the like, basic amines, or basic amino acids are also ideal in the form of pharmaceutically acceptable salts of compounds of formula (I) used in the present disclosure. Moreover, alcohols, carboxylic acids, or the like having a carbon number of 1 to 10, preferably methyl alcohol, ethyl alcohol, acetic acid, or propionic acid, and the like are ideal in the form of pharmaceutically acceptable esters of compounds of formula (I) used in the present disclosure. Moreover, water or the like is ideal in the form of a pharmaceutically acceptable solvate of compounds of formula (I) used in the present disclosure.

The description of specific examples of compounds of formula (I) such as SMTP-7 above also includes salts, esters, solvates, and other forms thereof.

### (Uses of Compounds of Formula (I))

Uses of compounds of formula (I) include a use for treating or preventing cerebral hemorrhage. In other words, according to the present disclosure, a compound of the formula (I) or a pharmaceutically acceptable salt, ester, or solvate thereof for use in treatment or prevention of cerebral hemorrhage is also provided. Details of the method of use and the like in this case are the same as the details of the method of use and the like in the method of treatment or prevention of cerebral hemorrhage described below, and the preferred aspect is also the same. Compounds of formula (I) may be used, for example, in methods of treating or preventing cerebral hemorrhage described below. Compounds of formula (I) are effective not only in reducing or eliminating hematomas caused by cerebral hemorrhage, but also in reducing or eliminating edema. Since hematoma and edema are different phenomena, it is a surprising effect that compounds of formula (I) have an effect on both hematoma and edema.

According to the present disclosure, a use of a compound of the formula (I) or a pharmaceutically acceptable salt, ester, or solvate thereof in production of a pharmaceutical for treatment or prevention of cerebral hemorrhage is also provided.

In one embodiment, a compound of formula (I) or a pharmaceutically acceptable salt, ester, or solvate thereof is administered to a subject for treatment or prevention of cerebral hemorrhage without cerebral infarction (in other words, not associated with cerebral infarction).

### (Methods of Treatment or Prevention of Cerebral Hemorrhage)

A method of treatment or prevention of cerebral hemorrhage of the present disclosure is a method of treatment or prevention of cerebral hemorrhage in a subject, including administering to the subject having cerebral hemorrhage or having a risk of onset of cerebral hemorrhage a compound of formula (I) or a pharmaceutically acceptable salt, ester, or solvate thereof at an amount effective for treating or preventing cerebral hemorrhage.

By using a treatment or prevention method of the present disclosure, effects of, for example, suppression of exacerbation of cerebral hemorrhage, reducing or alleviating the symptoms of cerebral hemorrhage, or inhibiting the onset, reducing the risk of onset, or delay of the onset of cerebral hemorrhage, or the like can be obtained. More specifically, in the brain, effects such as reduction or elimination of a once-occurred hematoma, reduction or elimination of once-occurred edema, and recovery of brain function. Note that here, "subject having cerebral hemorrhage" includes not only a subject with ongoing cerebral hemorrhage but also a subject whose cerebral hemorrhage has stopped, but has some injury (for example, presence of hematoma, presence of edema, damage to brain function, and the like) resulting from cerebral hemorrhage.

In the present disclosure, "treatment" is a term encompassing controlling exacerbation and reduction or alleviation of symptoms, so long as symptoms are ameliorated or suppressed.

In the present disclosure, "prevention" means inhibition of onset, reduction of the risk of onset, delay of onset, and the like.

Compounds of formula (I) (including pharmaceutically acceptable salts, esters, or solvates thereof; the same applies hereinafter) may be used to treat subjects for whom symptoms of cerebral hemorrhage have been found (including subjects who have damage due to cerebral hemorrhage even though the hemorrhage itself has stopped, as described above), and to prevent cerebral hemorrhage in subjects who have not actually developed a cerebral hemorrhage but are at risk of onset of cerebral hemorrhage (particularly subjects for whom onset of cerebral hemorrhage is predicted).

Compounds of formula (I) may be used to eliminate symptoms caused by cerebral hemorrhage, suppress the progression of the above symptoms, or relieve the above symptoms. However, these effects are not to be construed as limiting, as they may be compounded depending on the timing of use or the symptoms at the time of use.

Examples of when symptoms due to cerebral hemorrhage are found, or when symptoms due to cerebral hemorrhage are predicted to occur, include during or after treatment for intracerebral hemorrhage, subarachnoid hemorrhage, or the like. Alternatives include a period after administration of thrombolytic agents such as alteplase and urokinase which generally have hemorrhaging as a side effect, when cerebral hemorrhage due to administration of these thrombolytic agents is predicted. Use prophylactically is also possible during these periods. The risk of onset of cerebral hemorrhage may also be determined by brain imaging, blood tests, and other tests for physiological indicators. For subjects determined to have such a risk, compounds of formula (I) may be prophylactically administered even before the actual manifestation of cerebral hemorrhage.

Cerebral hemorrhage is a condition in which a blood vessel in the brain ruptures, causing a hemorrhage in the brain. Blood leaking from blood vessels forms a hematoma, as well as edema in the area surrounding the hemorrhage. Hematomas and edema compress normal brain tissue and impair brain function. Compounds of formula (I) are effective not only in reducing or eliminating hematomas, but also in reducing or eliminating edema. Since hematoma and edema are different phenomena, it is a surprising effect that compounds of formula (I) have an effect on both hematoma and edema.

Cerebral hemorrhage can be broadly divided into intracerebral hemorrhage and subarachnoid hemorrhage based on the location at which the hemorrhage occurred. Subarachnoid hemorrhage is essentially caused by a ruptured cerebral aneurysm. Intracerebral hemorrhage refers to hemorrhages in various areas of the brain, including examples such as putaminal hemorrhages, thalamic hemorrhages, subcortical hemorrhages, cerebellar hemorrhages, and pontine hemorrhages. Compounds of formula (I) are effective against both these intracerebral hemorrhages and subarachnoid hemorrhages.

Conventionally, surgical measures such as neck clipping and endovascular surgery have been used for subarachnoid hemorrhages, and surgical measures such as hematoma evacuation surgery have been used for intracerebral hemorrhages. It is surprising that compounds of formula (I) are able to treat or prevent cerebral hemorrhage by medication.

Cerebral hemorrhage is a condition essentially unlike cerebral infarction, and in one embodiment, a compound of formula (I) is administered to a subject for treatment or prevention of cerebral hemorrhage without cerebral infarction (in other words, not associated with cerebral infarction). The treatment or prevention methods of the present disclosure are applicable to both intracerebral hemorrhage and subarachnoid cerebral hemorrhage.

Although it depends on the type of coexisting compounds, the severity of the cerebral hemorrhage, and the like, a single effective dose for an adult of a compound of formula (I) (total amount of a compound of formula (I)) is preferably administration at 0.001 to 100 mg/kg, and more preferably administration at 0.01 to 30 mg/kg. A number of administrations of a compound of formula (I) is not particularly limited, and may be used in a single administration, used in repeated administration, or used in continuous administration. The administration interval and administration period may be selected by a person skilled in the art according to clinical findings, imaging findings, blood findings, comorbidities, medical history, and the like.

When a compound of formula (I) is used in repeated administration, from the perspective of continuous affected area contact with a compound of formula (I), an aspect of continuous administration of 1 to 24 hours per day is also preferable.

The method of administration is not particularly limited, and various routes of administration may be selected, such as intravenous administration, intraarterial administration, intraperitoneal administration, subcutaneous administration, intramuscular administration, oral administration, and intracerebral administration.

Intracerebral administration may be performed by, for example, using a microsyringe fitted with a microneedle, inserting the microneedle into a guide cannula implanted in the brain, and injecting a drug solution into a predetermined brain site. Intracerebral administration is preferable from the perspective of dose reduction.

The drug of the present disclosure may be used without limitation for use in humans. The drug may also be used in other applicable targets including non-human animals, such as livestock such as cattle, horses, and sheep, and pets such as dogs, cats, and monkeys.

### <Concomitant Use with Other Drugs>

Compounds of formula (I) may be used alone or together with at least one or more other drugs (for example, antihypertensive agents).

### <Pharmaceutical Composition>

Compounds of formula (I) may be used in a pharmaceutical composition. That is, a pharmaceutical composition containing a compound of formula (I) and at least one of a pharmaceutically acceptable carrier and a formulation additive is provided. The uses, dosage, administration method, and the like of the pharmaceutical composition are the same as uses of the drug of the present disclosure, and can be used in, for example, treatment or prevention methods of cerebral hemorrhage of the present disclosure. That is,
• A method of treating cerebral hemorrhage in a subject, including administering to the subject having a cerebral hemorrhage a pharmaceutical composition of the present disclosure in an amount effective for treating the cerebral hemorrhage; and
• A method of preventing cerebral hemorrhage in a subject, including administering to the subject having a risk of onset of cerebral hemorrhage a pharmaceutical composition of the present disclosure in an amount effective for preventing cerebral hemorrhage are provided.

The types of carriers and formulation additives that are optionally contained in the pharmaceutical composition are not particularly limited. A pharmaceutical composition of the present disclosure may be formulated using a compound of formula (I) of the present disclosure and a pharmaceutically acceptable solid carrier (for example, gelatin or lactose) or liquid carrier (for example, water, physiological saline, or a glucose aqueous solution).

### Examples

Several examples of the present disclosure are described below, but examples of the present disclosure are not limited thereto. Note that unless specified otherwise, "%" is based on mass.

### <Preparation of SMTP-7>

SMTP-7 was obtained using the method taught in JP 2004-224738 A to purify from a culture obtained when L-omithine was added as an additive organic amino compound to a culture medium of *stachybotrys microspora* IFO 30018 strain. A 50 mg/mL solution was prepared by adding 0.3 N (0.3 mol/L) NaOH solution and physiological saline (0.9% NaCl) to a dried solid of SMTP-7 obtained by purification. Then, 0.3 N (0.3 mol/L) HCl solution and physiological saline were used to adjust the concentration of SMTP-7 to a concentration of 10 mg/mL and a slightly alkaline pH, and the solution was filtration sterilized, subdivided, and cryopreserved at -30°C. SMTP-7 was used diluted using physiological saline as needed.

SMTP-7 used as a test solution in the following experiment with the above cryopreserved solution dissolved to 2 mg/mL in physiological saline solution immediately before testing.

Each drug used in the experiments was diluted using physiological saline as needed.

### <Example 1>

### Rat Preparation

Eight to 10 week old male SD rats (250 to 300 g body weight) were kept under a 12-hour light/dark cycle and a temperature of 22°C ± 1°C. The rats were raised in plastic cages and given free access to water and feed. The number of rats used in each experiment was n = 4 unless otherwise indicated. Moreover, a component other than those listed in the injection solution was physiological saline.

The rats were anesthetized with isoflurane (1.5% isoflurane + 66% NO + 33% O₂) and then injected with collagenase from *clostridium histolyticum* (type IV, made by Sigma-Aldrich) at a concentration of 0.25 U/µL at a position 3 mm lateral from the center of the brain, at bregma level in the anteroposterior direction and 5 mm behind the stria vascularis using a syringe to inject for 5 minutes at a rate of 0.2 µL/min, then the syringe needle was held at that position for 10 minutes, and then slowly withdrawn over another 10 minutes (0.5 mm per minute). Note that care was taken to prevent backflow during injection. In the following description, the day of this collagenase injection is referred to as day 0. This collagenase injection causes an intracerebral hemorrhage and the formation of a hematoma.

Twenty-four hours after the collagenase injection (that is, day 1 after the collagenase injection), the test solution (physiological saline, 2 mg/mL solution of SMTP-7) was injected through the same injection track as the collagenase injection at a rate of 0.2 µL/min over 10 minutes. A test group without injection of the test solution was also included. Care was taken to prevent backflow during injection. As during the collagenase injection, the syringe needle was held in that position for 10 minutes after injection and then slowly withdrawn over another 10 minutes (0.5 mm per minute).

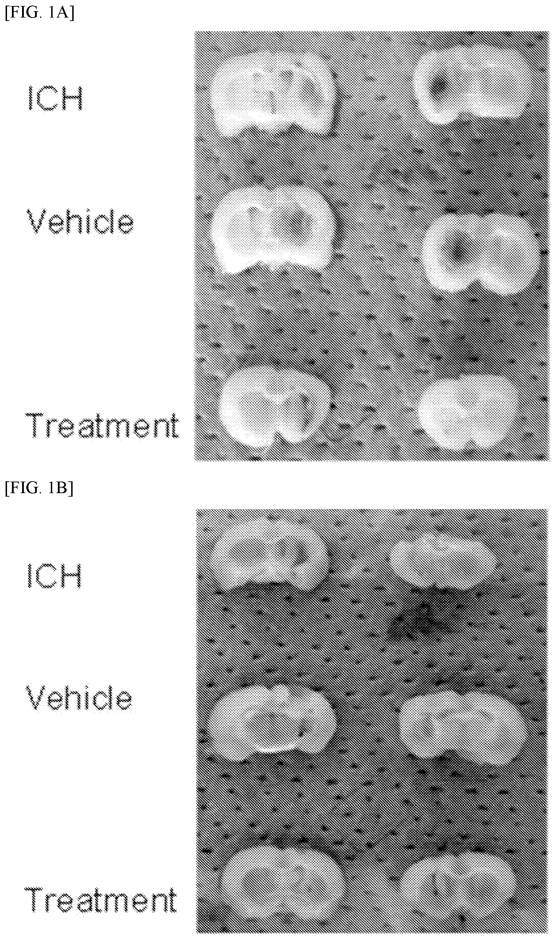

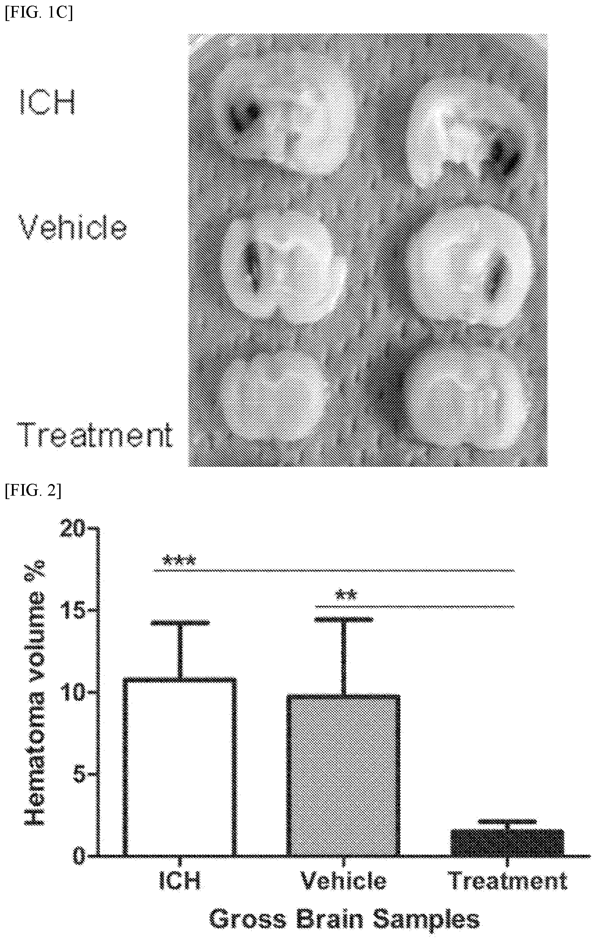

On day 7 after the collagenase injection, the rats were sacrificed and 3 mm thick sections of brain were prepared. A 4% paraformaldehyde solution in 0.1 M PBS (pH 7.4) was used for fixation during section preparation. Micrographs of these sections are illustrated in FIG. 1A to FIG. 1C. In FIG. 1A to FIG. 1C, "ICH" represents sections of individuals who received a collagenase injection but no test solution injection, "Vehicle" represents sections of individuals who received a physiological saline injection instead of a collagenase injection, and "Treatment" represents sections of individuals who received a test solution containing SMTP-7. This notation is the same in the following figures.

Moreover, the ratio of volume of hematoma to the volume of the entire brain was also measured using Imaged. The results are shown in FIG. 2. Note that in the graphs of the present disclosure, the vertical axis is a value of the ratio of the volume of hematoma to the volume of the entire brain sample expressed as a percentage, ** indicates that the p-value by t-test for significant difference is less than 0.01, and *** indicates that the p-value by t-test for significant difference is less than 0.001 (the meaning of ** and *** is the same below). Moreover, the bars in the graphs represent standard deviations. As shown in FIG. 1A to FIG. 1C and FIG. 2, individuals injected with the test solution containing SMTP-7 showed a marked reduction in hematoma.

### <Example 2>

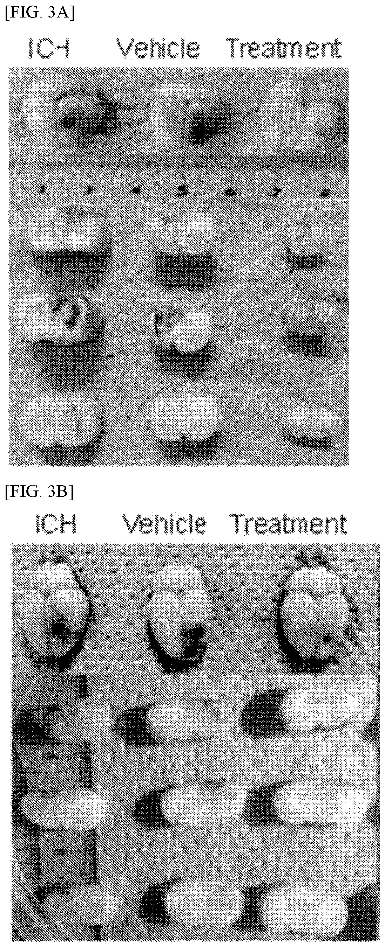

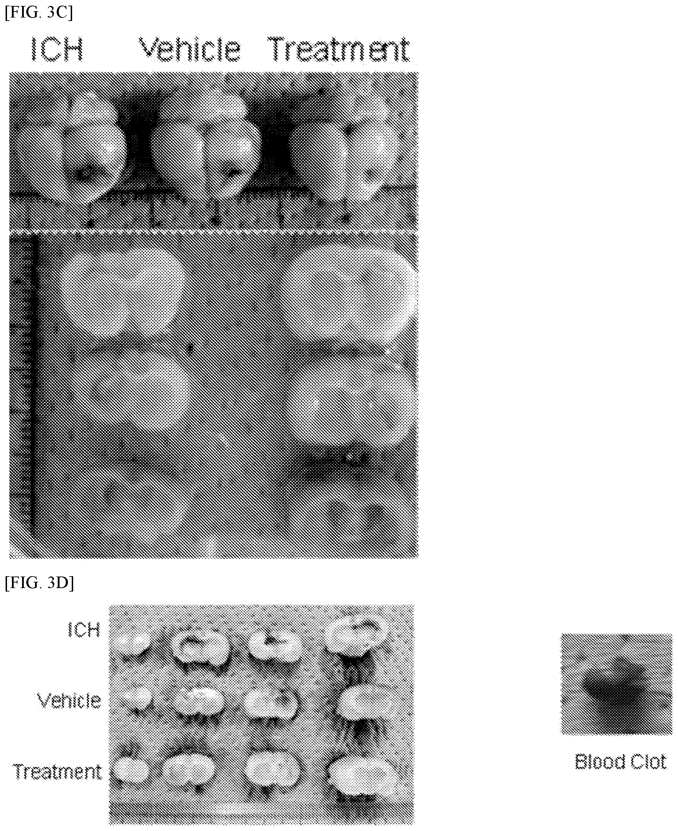

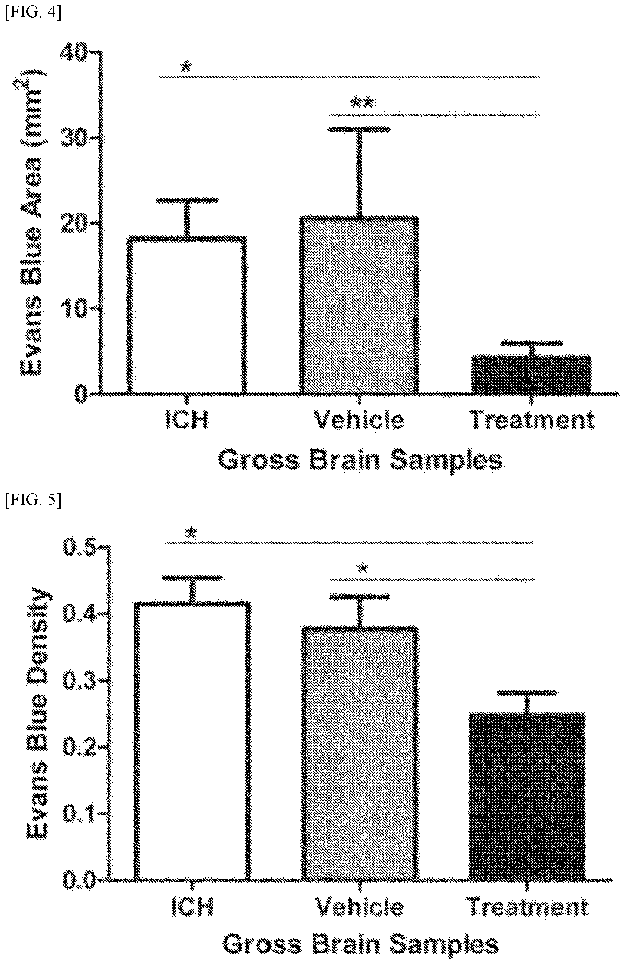

The same experiment as in example 1 was performed again. However, in example 2, physiological saline containing 2% Evans blue was injected into the tail vein 1 hour before sacrifice (day 7) at a volume of 4 mL per 1 kg of body weight. Evans blue has a property of binding to albumin in the blood, and as a result of collagenase injection, the hematoma is stained because the blood-brain barrier is ruptured. Micrographs of whole brains and 3 mm thick sections are illustrated in FIG. 3A to FIG. 3D, and the total area (mm²) of Evans blue-stained region and staining density determined from the sections using Imaged are shown in FIG. 4 and FIG. 5. FIG. 3D also illustrates the appearance of Evans blue staining of blood clots. In FIG. 4, the vertical axis indicates the area (mm²) of Evans blue-stained regions in whole brain samples. Moreover, * indicates that the p-value in the t-test for significant difference is less than 0.05, and ** indicates that the p-value in the t-test for significant difference is less than 0.01 (the meaning of * and ** is the same below). In FIG. 5, the vertical axis represents the concentration value of Evans blue expressed as absorbance at a wavelength of 620 nm. As shown in FIG. 3A to FIG. 3D, FIG. 4, and FIG. 5, individuals injected with the test solution containing SMTP-7 showed a marked reduction in hematoma.

### <Example 3>

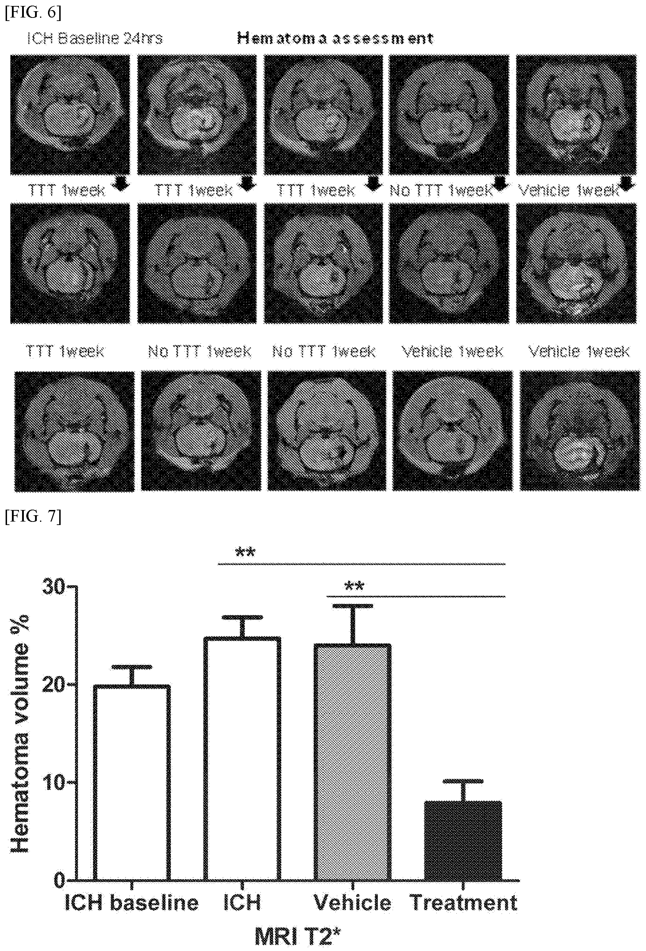

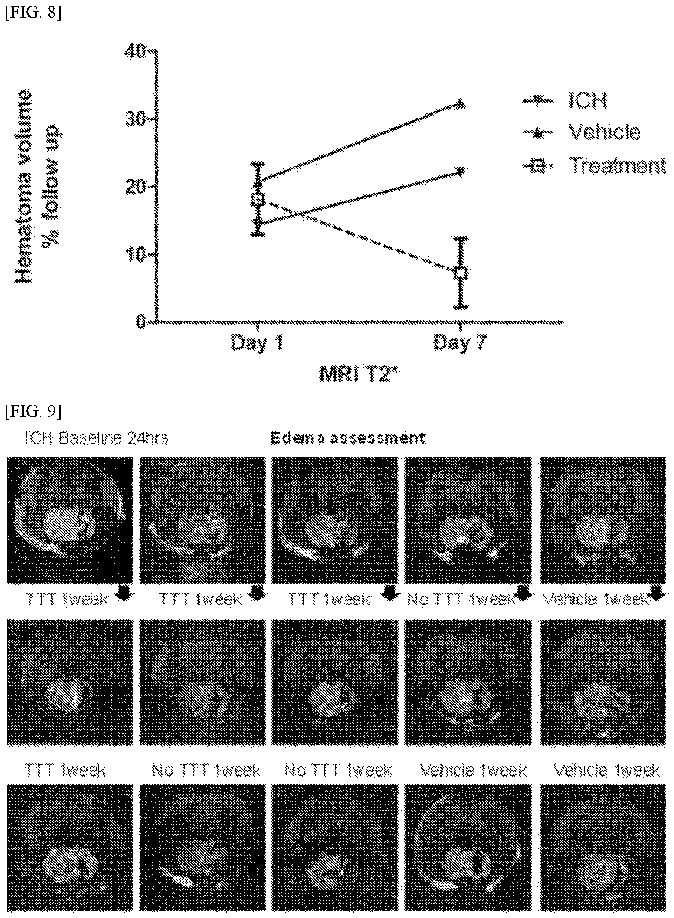

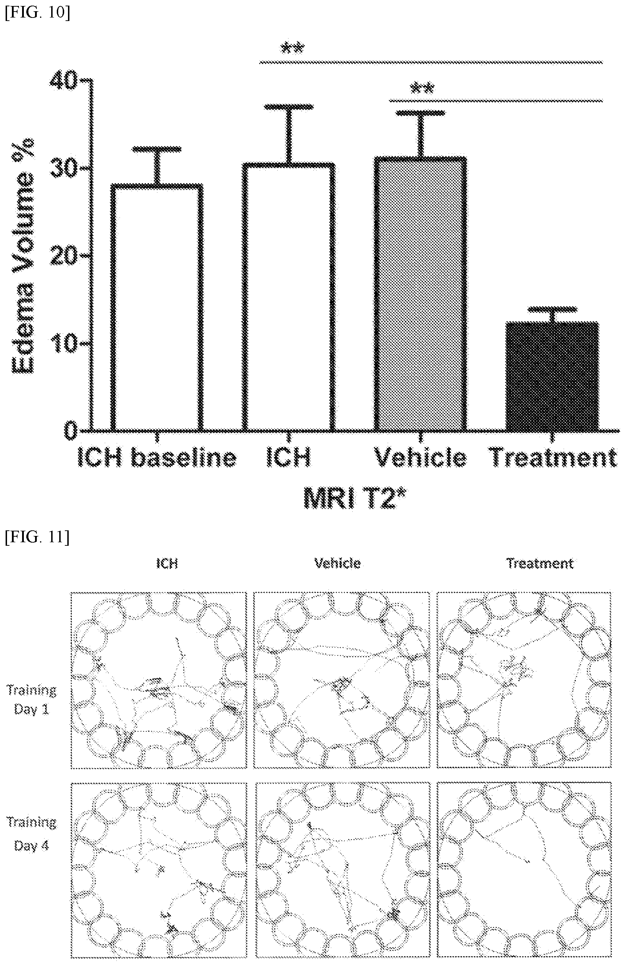

Collagenase injection and test solution injection were performed as in example 1, and rat brains were measured by small animal MRI on days 1 and 7 after collagenase injection (PharmaScan made by Bruker BioSpin). Measurement was performed using conditions of magnetic field 7T, echo time (TE) = 4 msec, repetition time (TR) = 800 msec, 256 × 256 matrix, slice thickness 0.8 mm, and average scan time about 5 min 7 sec, to obtain T2-weighted images. For MRI measurements, rats were anesthetized with 2.5% to 3% isoflurane + NO + O₂ and measurements were performed while alive. The results are illustrated in FIG. 6. In FIG. 6, "ICH Baseline 24hrs" represents an image of a rat brain on day 1 after collagenase injection, "TTT 1week" represents an image of a rat brain on day 7 after injection of the test solution containing SMTP-7, "No TTT 1week" represents an image of a rat brain on day 7 without injection of the test solution, and "Vehicle 1week" represents an image of a rat brain on day 7 after injection of physiological saline instead of the test solution. As illustrated in FIG. 6, "TTT 1week" illustrates four specimens, and "No TTT 1week" and "Vehicle 1week" each illustrate three specimens. The volume of hematomas was measured based on these MRI measurements using Imaged. The hematoma volume on day 7 after collagenase injection is shown in FIG. 7, and the change in hematoma volume from the hematoma volume on day 1 after collagenase injection is shown in FIG. 8. In FIG. 7, the vertical axis shows the volume ratio of hematoma in the T2-weighted MRI image as a percentage. Moreover, "ICH Baseline" indicates a rat on day 1 after collagenase injection, "ICH" indicates a rat on day 7 without injection of the test solution after collagenase injection, "Vehicle" indicates a rat on day 7 after injection of physiological saline instead of the test solution, and "Treatment" indicates a rat on day 7 after injection of the test solution containing SMTP-7. In FIG. 8, the horizontal axis represents the number of days elapsed after collagenase injection, and the vertical axis shows the ratio (%) of hematoma volume in brain volume at each time point. Furthermore, perihematoma edema and the resulting mass effect (mass effect) were observed. The results are illustrated in FIG. 9. In FIG. 9, "ICH Baseline 24hrs" represents an image of a rat brain on day 1 after collagenase injection, "TTT 1week" represents an image of a rat brain on day 7 after injection of the test solution containing SMTP-7, "No TTT 1week" represents an image of a rat brain on day 7 without injection of the test solution, and "Vehicle 1week" represents an image of a rat brain on day 7 after injection of physiological saline instead of the test solution. As illustrated in FIG. 9, "TTT 1week" illustrates four specimens, and "No TTT 1week" and "Vehicle 1week" each illustrate three specimens. Moreover, the volume of cerebral edema was determined based on these observation results using Imaged. Cerebral edema volume on day 7 after collagenase injection is shown in FIG. 10. In FIG. 10, the vertical axis shows the ratio (%) of cerebral edema volume in brain volume, "ICH Baseline" indicates a rat on day 1 after collagenase injection, "ICH" indicates a rat on day 7 without injection of the test solution after collagenase injection, "Vehicle" indicates a rat on day 7 after injection of physiological saline instead of the test solution, and "Treatment" indicates a rat on day 7 after injection of the test solution containing SMTP-7. As shown in FIG. 6 to FIG. 10, individuals injected with the test solution containing SMTP-7 showed a marked reduction in both hematoma and edema in the brain.

### <Example 4>

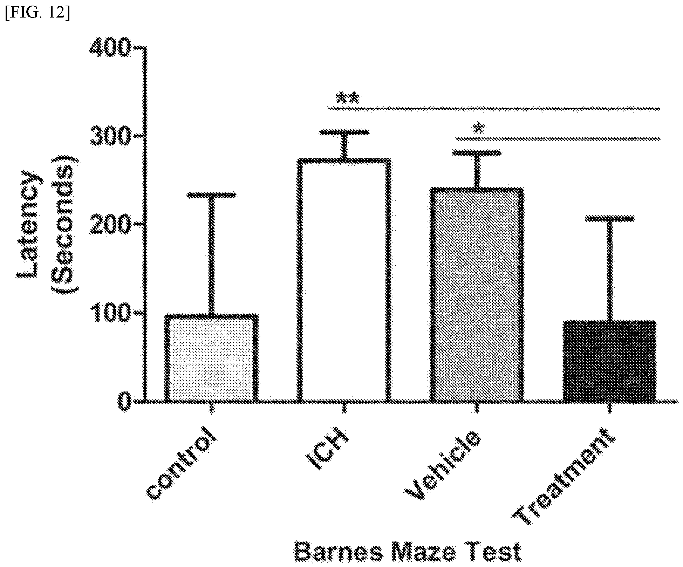

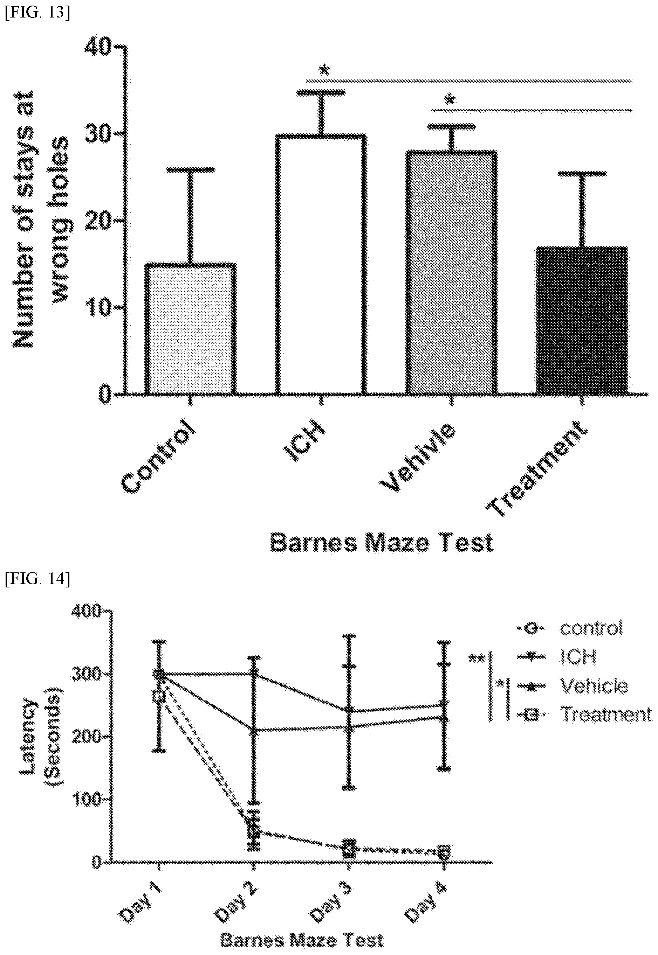

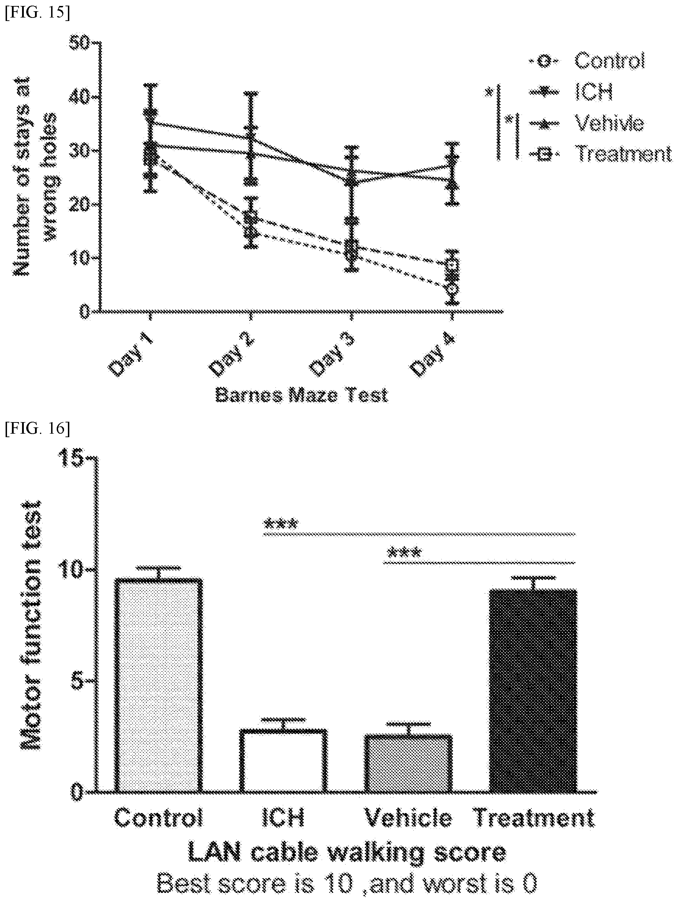

Collagenase injection and test solution injection were performed as in example 1, and a Barnes maze test (Barnes maze test) was performed on day 7 after collagenase injection. This test allows for assessment of spatial learning and memory. The test was performed using a gray acrylic platform with a diameter of 122 cm, and 18 holes were arranged on the periphery of the acrylic platform. Rat behavior was observed by placing a camera above the rats' heads. The evaluation was performed based on the time it took to locate the escape box. Training was performed by performing the test twice daily and the test was performed up to day 10 after collagenase injection. In each test, the rats started from a start box provided in the center of the maze, stayed in the start box for the first 30 seconds, and then were allowed to explore the maze freely. Once the rat reached the escape box, the rat was allowed to stay in the escape box for 30 seconds before being returned to the raising cage. If the rat did not reach the escape box within the maximum test time of 300 seconds, the rat was picked up and allowed to stay in the evacuation box for 30 seconds before being returned to the raising cage. The time it took to reach the escape box and the number of holes where the rats incorrectly stayed at were evaluated. Note that in example 4, rats that had not received any treatment were also tested as controls. The path of rats on day 7 (Training Day 1 in the figure) and day 10 (Training Day 4 in the figure) after collagenase injection are illustrated in FIG. 11. In FIG. 11, "ICH" indicates a rat without injection of the test solution after collagenase injection, "Vehicle" indicates a rat after injection of physiological saline instead of the test solution, and "Treatment" indicates a rat after injection of the test solution containing SMTP-7. The time required to reach the escape box and the number of incorrect holes stayed on day 10 after collagenase injection are shown in FIG. 12 and FIG. 13. In FIG. 12 to FIG. 15, "ICH" indicates a rat without injection of the test solution after collagenase injection, "Vehicle" indicates a rat after injection of physiological saline instead of the test solution, "Treatment" indicates a rat after injection of the test solution containing SMTP-7, and "control" indicates a healthy rat without collagenase injection. Moreover, the chronological changes in the time required and the number of incorrect holes on each day from day 7 to day 10 after collagenase injection, that is, the degree of learning, are shown in FIG. 14 and FIG. 15. Day 1 in FIG. 14 and FIG. 15 indicates day 7 after collagenase injection. As shown in FIG. 11 to FIG. 15, individuals injected with the test solution containing SMTP-7 showed a marked improvement in learning ability.

### <Example 5>

Collagenase injection and test solution injection were performed as in example 1, and the motor function of rats was evaluated by having the rats walk on a local area network (LAN) cable. The rats were placed on a LAN cable at a height of 60 cm from the floor along the length of the LAN cable and observed for 60 seconds for hemiplegia, posture on the cable, activity, and resilience. The following criteria were used to distribute the scores for each item, and total scores (0 to 10) were obtained.

### <Right-Side Paralysis>

[Forelegs]
0 points If there is right-side paralysis
1 point If there is no right-side paralysis
[Hind Legs]
0 points If there is right-side paralysis
1 point If there is no right-side paralysis

### <Posture on Cable>

0 points Cannot even hang on for 10 seconds.
1 point Can hang for 10 seconds or more, but cannot maintain posture for crossing the LAN cable for 30 seconds
2 points Can maintain posture for crossing the LAN cable for 30 seconds or more, but cannot maintain posture along the LAN cable for 30 seconds
3 points Can maintain posture along the LAN cable for 30 seconds or more

### <Activity>

[Continuity of Activity]
0 points Unable to remain active for 10 seconds
1 point Able to remain active for 10 seconds or more
[Restoration of Posture]
0 points Unable to restore posture when balance is lost
1 point Able to restore posture when balance is lost
[Travel Distance]
0 points Cannot move 10 cm
1 point Able to move 10 cm or more

### <Resilience>

0 points Falls from LAN cable
2 points Does not fall from LAN cable

The results are shown in FIG. 16. The vertical axis in FIG. 16 shows the total points of the above. Note that in example 5, rats that had not received any treatment were also tested as controls. In FIG. 16, "ICH" indicates a rat without injection of the test solution after collagenase injection, "Vehicle" indicates a rat after injection of physiological saline instead of the test solution, "Treatment" indicates a rat after injection of the test solution containing SMTP-7, and "control" indicates a healthy rat without collagenase injection. As shown in FIG. 16, individuals injected with the test solution containing SMTP-7 showed a marked improvement in motor ability.

### <Example 6>

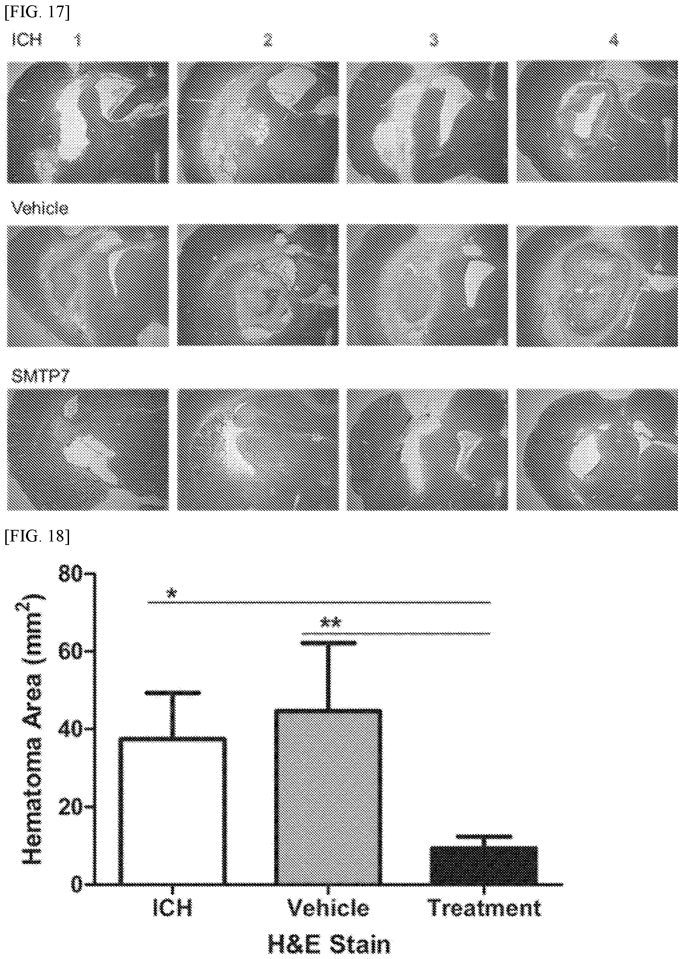

Collagenase injection and test solution injection were performed as in example 1, and brain tissue specimens were obtained on day 7 after collagenase injection. Specifically, rats were deeply anesthetized using isoflurane, perfused using physiological saline, and further perfused using 4% formaldehyde in 0.1 M phosphate buffered saline (PBS; pH 7.4). The brains were removed and postfixed overnight at 4°C with the above PBS solution containing 4% formaldehyde as the fixative solution and stored in 10%, 20%, and 30% sucrose in PBS until the tissue settled. Sections of approximately 2 mm thickness were obtained by cutting in the coronal plane using a brain slicer (made by Harvard Apparatus), embedded in an optimal cutting temperature (OCT) compound, frozen using liquid nitrogen, and cut into 10 µm to 12 µm thick sections using a cryostat microtome. Sections corresponding to median line-3 mm at bregma level were hematoxylin-eosin (HE) stained. Micrographs of this are illustrated in FIG. 17. The display scale is 100 µm (the same in subsequent micrographs). In FIG. 17, "ICH" indicates a rat without injection of the test solution after collagenase injection, "Vehicle" indicates a rat after injection of physiological saline instead of the test solution, and "SMTP-7" indicates a rat after injection of the test solution containing SMTP-7. The hematoma areas (mm²) on these sections measured using Imaged are shown in FIG. 18. The vertical axis in FIG. 18 represents the area (mm²) of the hematoma region identified by HE staining. Moreover, "ICH" indicates a rat without injection of the test solution after collagenase injection, "Vehicle" indicates a rat after injection of physiological saline instead of the test solution, and "Treatment" indicates a rat after injection of the test solution containing SMTP-7. As shown in FIG. 17 and FIG. 18, individuals injected with the test solution containing SMTP-7 showed a marked reduction in hematoma.

### <Example 7>

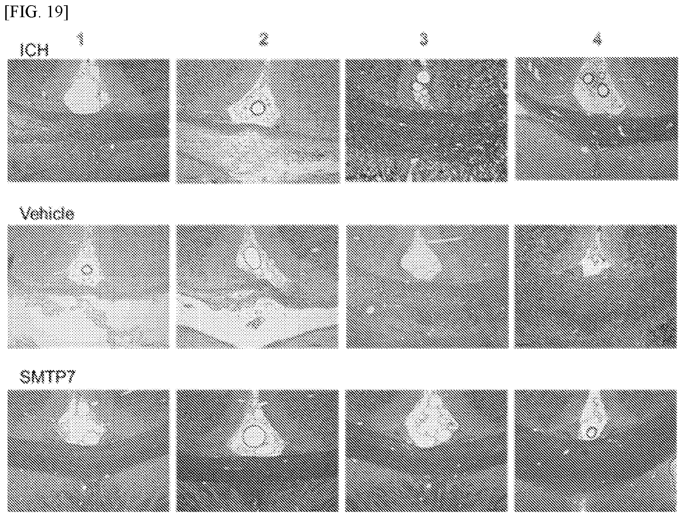

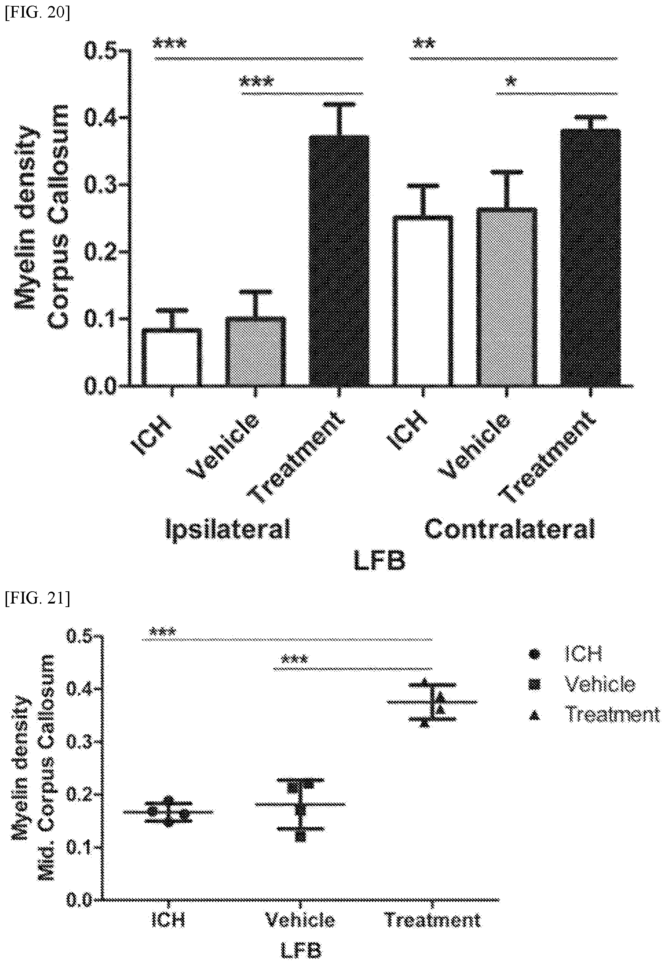

Ten µm thick sections prepared as in example 6 were double-stained with Luxol fast blue and Cresyl violet to observe white matter lesions (demyelination) inside of the corpus callosum. Micrographs of this are illustrated in FIG. 19. In FIG. 19, "ICH" indicates a rat without injection of the test solution after collagenase injection, "Vehicle" indicates a rat after injection of physiological saline instead of the test solution, and "SMTP-7" indicates a rat after injection of the test solution containing SMTP-7. Moreover, myelin density in the white matter was also measured at a site ipsilateral to the collagenase injection position, a site contralateral to the collagenase injection position, and at a central site. The results are shown in FIG. 20 and FIG. 21. In FIG. 20, the vertical axis shows myelin density (mean value) in white matter. In FIG. 20, "Ipsilateral" represents ipsilateral to the collagenase injection and "Contralateral" represents contralateral to the collagenase injection (same below). In FIG. 21, the vertical axis shows myelin density (value for each solid) in central white matter. In FIG. 20 and FIG. 21, "ICH" indicates a rat without injection of the test solution after collagenase injection, "Vehicle" indicates a rat after injection of physiological saline instead of the test solution, and "Treatment" indicates a rat after injection of the test solution containing SMTP-7. As shown in FIG. 19 to FIG. 21, individuals injected with the test solution containing SMTP-7 showed marked suppression of hematoma lesions.

### <Example 8>

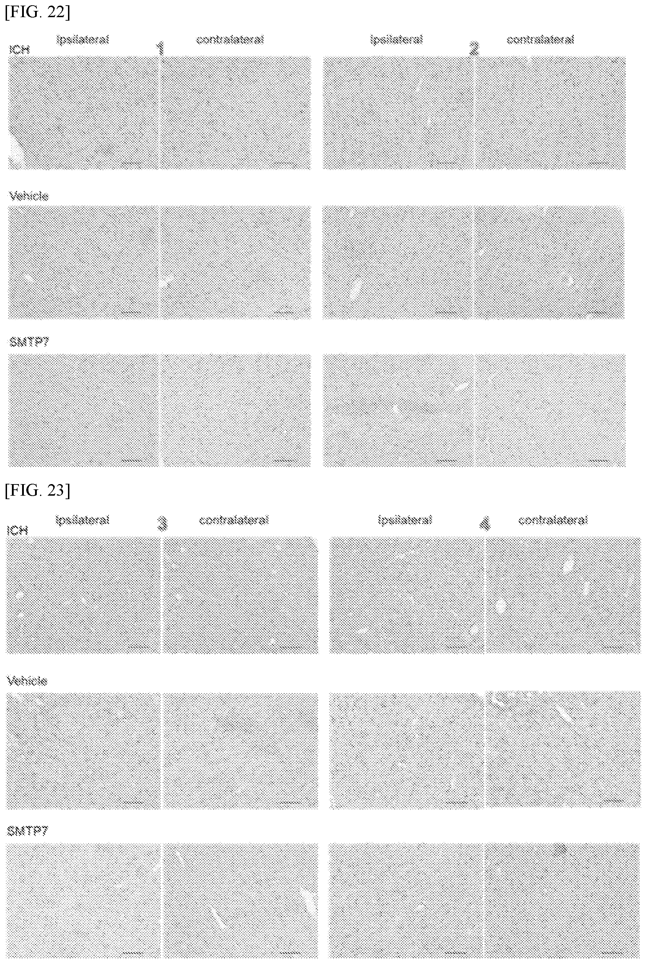

Ten µm to 12 µm thick sections (median line-3 mm at bregma level) prepared as in example 6 were treated using 10% bovine serum albumin (BSA) solution for 2 hours at room temperature to block nonspecific binding sites, and the blocked sections were incubated with an antibody against cleaved caspase-3 (Asp175) (9661 of Cell Signaling Technology). Furthermore, sections were exposed to biotinylated secondary antibodies (1:200; Vector Laboratory) and visualized using 0.01% diaminobenzidine tetrahydrochloride and 0.005% hydrogen peroxide in 50 mmol/L Tris-HCl (pH = 7.6). Micrographs of this are illustrated in FIG. 22 and FIG. 23. In FIG. 22 and FIG. 23, "ICH" indicates a rat without injection of the test solution after collagenase injection, "Vehicle" indicates a rat after injection of physiological saline instead of the test solution, and "Treatment" indicates a rat after injection of the test solution containing SMTP-7.

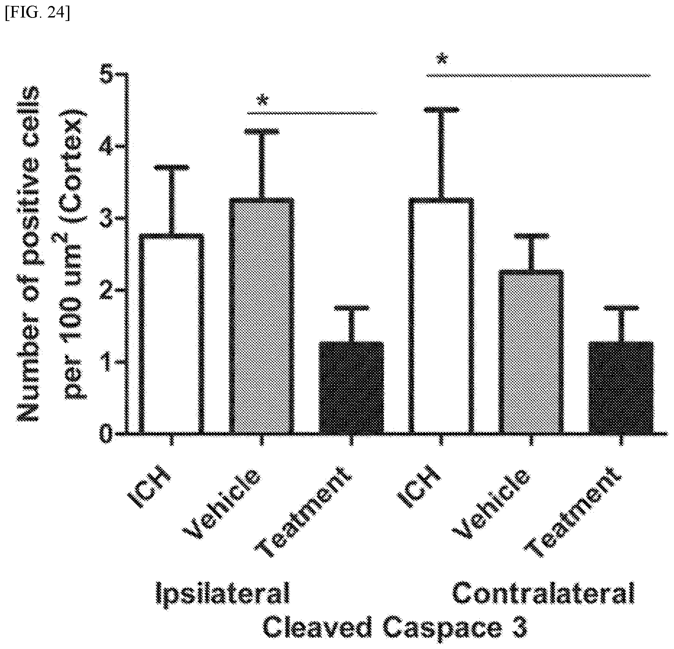

Furthermore, two 100 µm² regions of interest (ROI) were randomly selected in the cerebral cortex, and the number of immunopositive cells in each ROI was counted and averaged. In FIG. 24, the vertical axis represents a number of immunopositive cells per 100 µm² of ROI, "ICH" indicates a rat without injection of the test solution after collagenase injection, "Vehicle" indicates a rat after injection of physiological saline instead of the test solution, and "Treatment" indicates a rat after injection of the test solution containing SMTP-7. The results are shown in FIG. 24. As shown in FIG. 22 to FIG. 24, the number of cells expressing caspase-3, an indicator of apoptosis, was markedly reduced in individuals injected with the test solution containing SMTP-7, both ipsilateral (Ipsilateral) and contralateral (Contralateral).

### <Example 9>

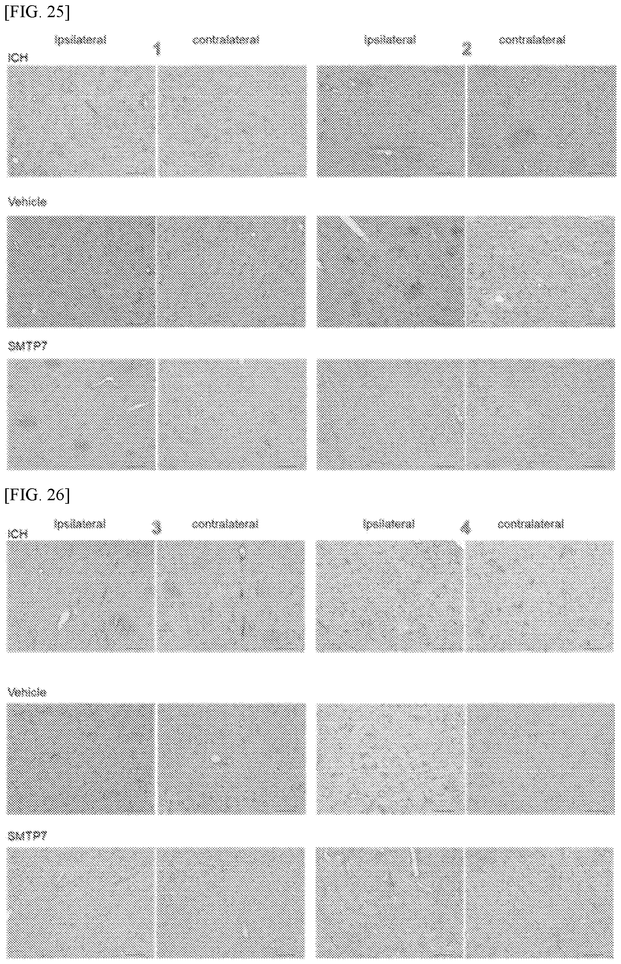

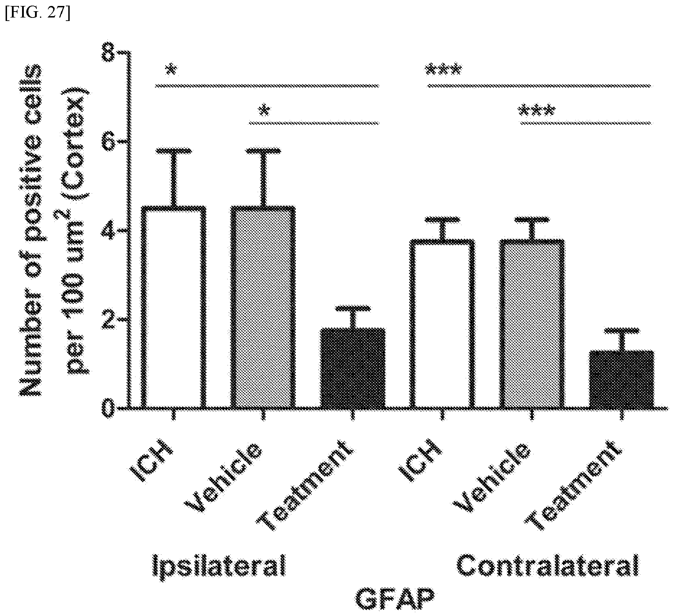

Except that anti-GFAP antibodies (axtrocyte; mouse mAb 3670; Cell Signaling Technology) were used instead of antibodies against cleaved caspase-3 (Asp175), an antibody staining test was performed as in example 8. Micrographs are illustrated in FIG. 25 and FIG. 26, and the number of immunopositive cells is shown in FIG. 27. The results of immunostaining using antibodies against GFAP, an astrocyte marker, as shown in FIG. 25 to FIG. 27, show that neuron damage is significantly suppressed in individuals injected with the test solution containing SMTP-7.

### <Example 10>

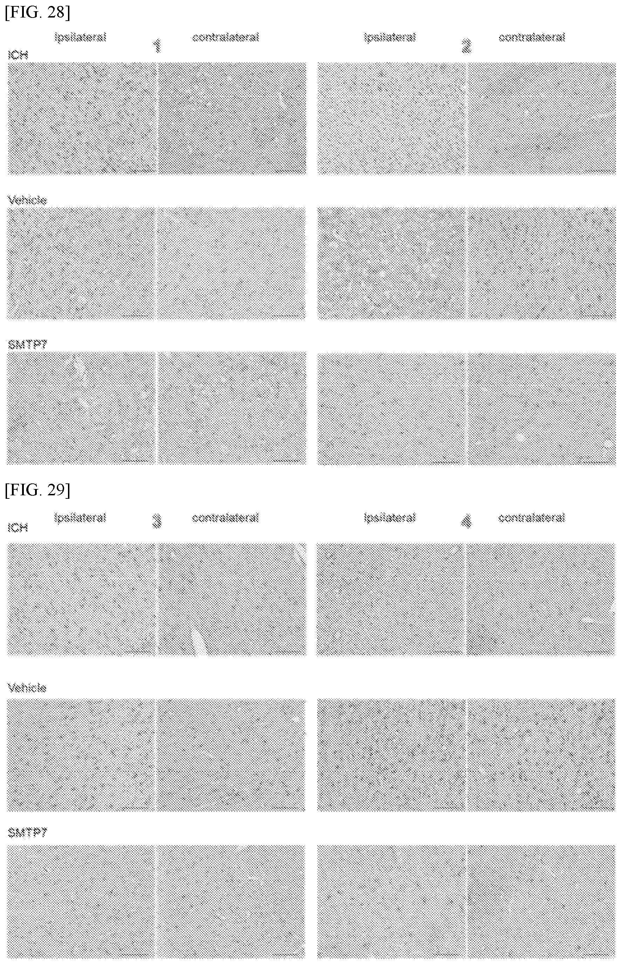

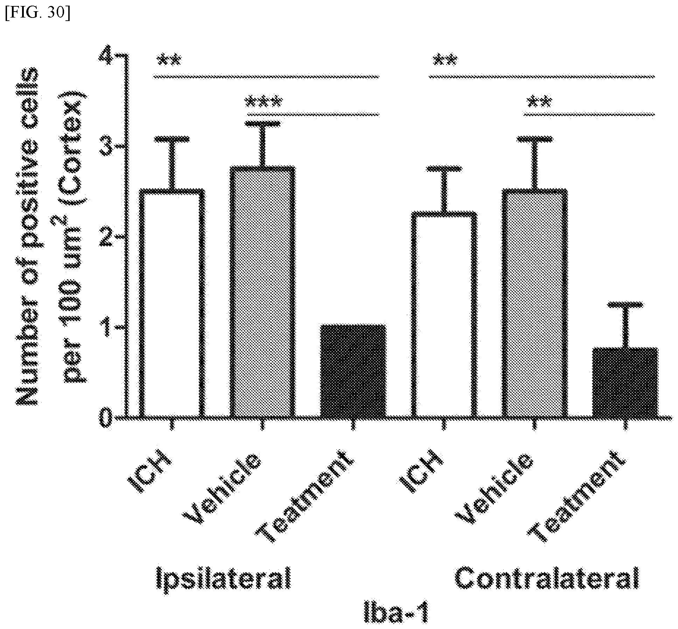

Except that anti-Iba-1 antibodies (019-19741; Wako; Cell Signaling Technology) were used instead of antibodies against cleaved caspase-3 (Asp175), an antibody staining test was performed as in example 8. Micrographs are illustrated in FIG. 28 and FIG. 29, and the number of immunopositive cells is shown in FIG. 30. The results of immunostaining using antibodies against Iba-1, a macrophage/microglia-specific marker, as shown in FIG. 28 to FIG. 30, show that inflammation is also significantly suppressed in individuals injected with the test solution containing SMTP-7.

The above results show that compounds of formula (I) have excellent treatment and prevention effects for cerebral hemorrhage.

All documents, patent applications, and technical standards described in the present specification are incorporated by reference herein to the same extent that individual documents, patent applications, and technical standards are specifically and individually noted as being incorporated by reference.

## Claims

1. A drug used for the treatment or prevention of cerebral hemorrhage, containing as an active ingredient a compound of formula (I) below or a pharmaceutically acceptable salt, ester or solvate thereof. 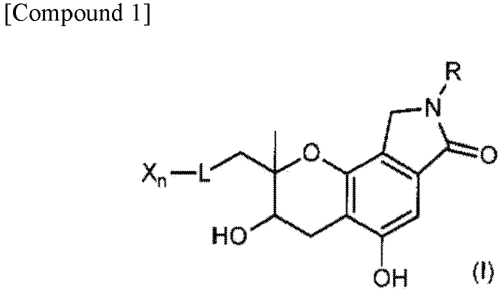 In formula (I), L represents an aliphatic hydrocarbon group having a carbon number of 4 to 10, X represents a hydroxy group or carboxy group, n represents an integer from 0 to 2, and R represents a hydrogen atom or a substituent having a molecular weight of 1,000 or less.

2. The drug according to claim 1, wherein the compound of the formula (I) or a pharmaceutically acceptable salt, ester, or solvate thereof is a compound of formula (IA) below or a pharmaceutically acceptable salt, ester, or solvate thereof. 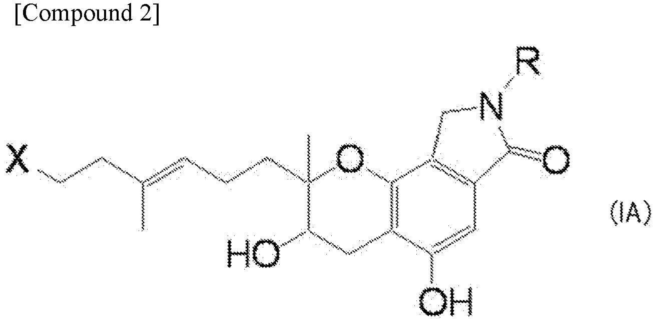 In formula (IA), X is -CHY-C(CH₃)₂Z, Y and Z are independently -H or - OH, or together form a single bond, and R represents a hydrogen atom or a substituent having a molecular weight of 1,000 or less.

3. The drug according to claim 1 or claim 2, wherein the compound of the formula (I) or a pharmaceutically acceptable salt, ester, or solvate thereof is a compound of formula (II) or formula (III) below or a pharmaceutically acceptable salt, ester, or solvate thereof. 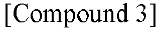 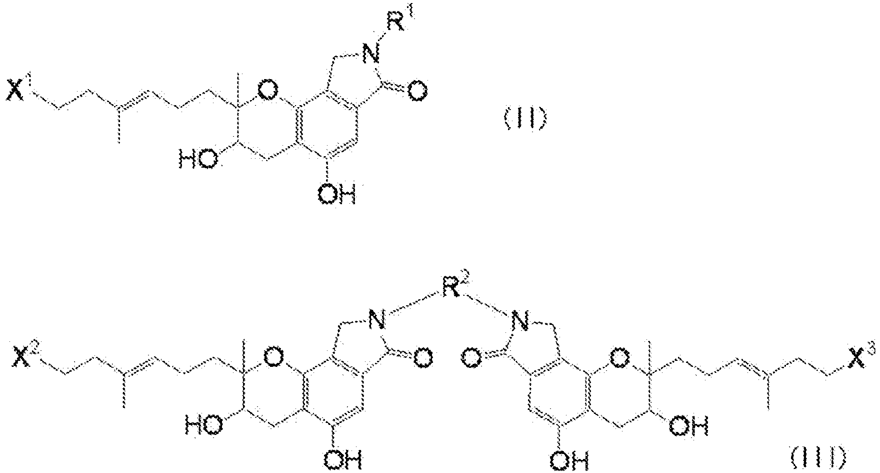 In formula (II) or formula (III), X¹, X², and X³ are independently -CHY-C(CH₃)₂Z, Y and Z are independently -H or -OH, or together form a single bond, and R¹ represents any one of (A) to (D) below.
(A) A residue having one amino group removed from an amino compound selected from a group composed of natural amino acids, D-bodies of natural amino acids, and compounds having at least one carboxy group in a natural amino acid or D-body of a natural amino acid replaced with a hydrogen atom, a hydroxy group, or a hydroxymethyl group (however, -(CH)₂-OH is excluded),
(B) an aromatic group having at least one selected from a group composed of a carboxy group, a hydroxy group, a sulfonic acid group, and a secondary amino group as a substituent or a part of a substituent, or an aromatic group containing a secondary amino group and that may contain a nitrogen atom,
(C) an aromatic amino acid residue of formula (II-1) below (in the formula, R³ is a respectively independent substituent which may be present or absent, and if present, represents a hydroxy group, a carboxy group or an alkyl group having a carbon number of 1 to 5, n represents an integer of 0 or 1, m represents an integer from 0 to 5, and * represents a binding site), and 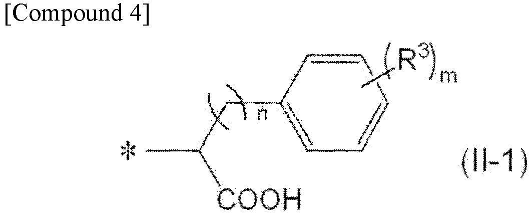
(D) a substituent represented by -L¹-L²-R⁴ (in the formula, L¹ represents a linking group which is an alkylene group having a carbon number of 1 to 4 having a carboxy group, L² represents a linking group represented by -NH-C(=O)- or -NH-C(=S)-NH-, R⁴ represents a 9-fluorenyl alkyloxy group having an alkyloxy group having a carbon number of 1 to 3, or a polyheterocyclic group of formula (II-2) below (in formula (II-2), and * represents a binding site)). 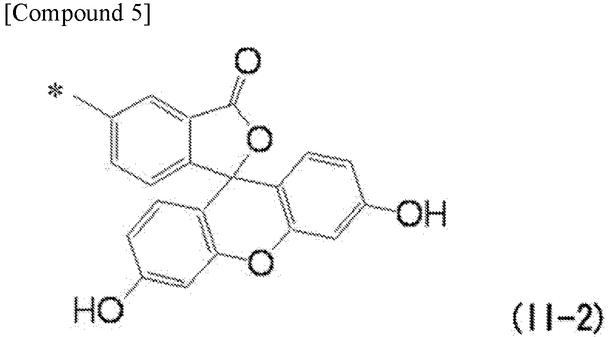 R² is a residue having two amino groups removed from an amino compound selected from a group composed of natural amino acids having 2 amino groups, D-bodies of natural amino acids having 2 amino groups, compounds having at least one carboxy group in a natural amino acid having 2 amino groups or a D-body of a natural amino acid having 2 amino groups replaced with a hydrogen atom, a hydroxy group, or a hydroxymethyl group, and compounds indicated by H₂N-CH(COOH)-(CH₂)ₙ-NH₂ (n is an integer from 0 to 9), and H₂N-CH(COOH)-(CH₂)ₘ-Sₚ-(CH₂)_{q}-CH(COOH)-NH₂ (m, p, and q are each independent integers from 0 to 9).

4. The drug according to any one of claim 1 to claim 3, wherein the compound of the formula (I) or a pharmaceutically acceptable salt, ester, or solvate thereof is a compound selected from a group composed of SMTP-0 below, SMTP-1 below, SMTP-4 below, SMTP-5D below, SMTP-6 below, SMTP-7 below, SMTP-8 below, SMTP-11 to 14 below, SMTP-18 to 29 below, SMTP-36 below, SMTP-37 below, SMTP-42 below, SMTP-43 below, SMTP-43D below, SMTP-44 below, SMTP-44D below, SMTP-46 below, and SMTP-47 below, or a pharmaceutically acceptable salt, ester, or solvate thereof. 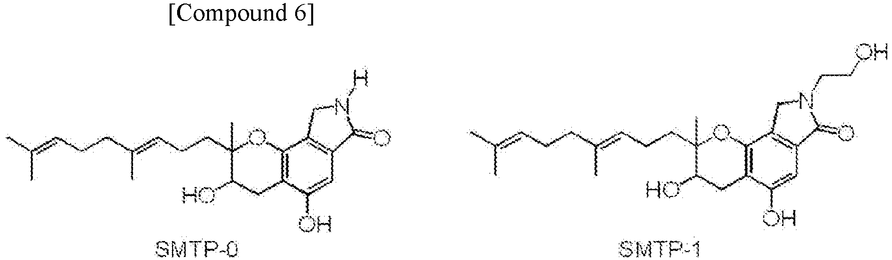 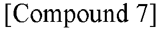 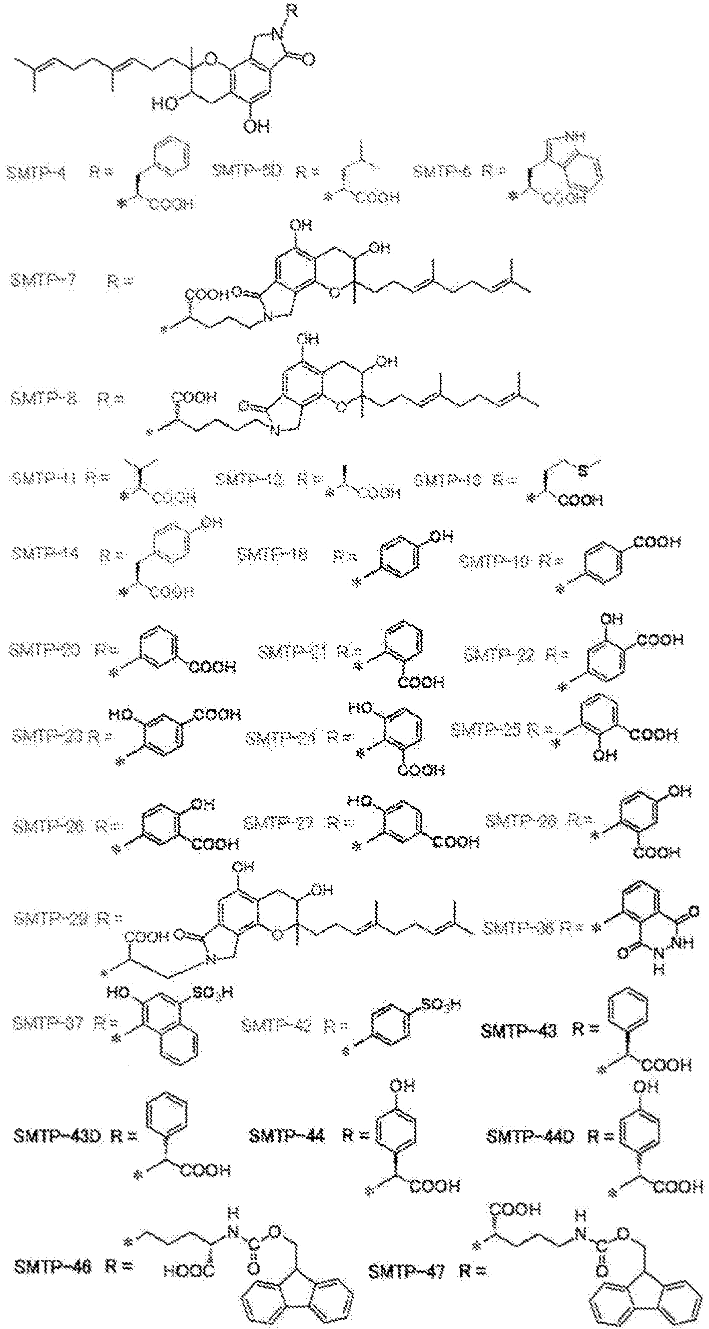 In the formula, * represents a binding site.

5. The drug according to claim 4, wherein the compound of the formula (I) or a pharmaceutically acceptable salt, ester, or solvate thereof is the SMTP-7 or a pharmaceutically acceptable salt, ester, or solvate thereof.

6. The drug according to any one of claim 1 to claim 5, wherein the cerebral hemorrhage is an intracerebral hemorrhage.

7. The drug according to any one of claim 1 to claim 6, which is intracerebrally administered.
